# EUROPÄISCHE PATENTSCHRIFT

(11) **EP 4 353 204 B1**
(45) Veröffentlichungstag und Bekanntmachung des Hinweises auf die Patenterteilung: **18.02.2026**
(21) Anmeldenummer: 23202705.2
(22) Anmeldetag: 10.10.2023
(51) Int. Cl.: A61F 5/02

(54) **RÜCKENORTHESE ZUM STABILISIEREN EINES RÜCKENS EINES PATIENTEN**
BACK ORTHOSIS FOR STABILIZING A BACK OF A PATIENT
ORTHÈSE DORSALE POUR STABILISER LE DOS D'UN PATIENT

(30) Priorität: 13.10.2022 DE 102022126748
(43) Veröffentlichungstag der Anmeldung: 17.04.2024
(73) Patentinhaber: Bort GmbH, 71384 Weinstadt-Benzach (DE)
(72) Erfinder: Barchen, Andreas, 72622 Nürtingen (DE); Strnad, Dieter, 73207 Plochingen (DE); Freihaut, Roman, 73614 Schorndorf (DE)
(74) Vertreter: Thum, Bernhard

(56) Entgegenhaltungen:
- DE-A1- 102006 043 845
- DE-A1- 102012 009 214
- DE-A1- 102015 107 582
- DE-U1- 202017 001 198
- US-A1- 2011 152 737
- US-A1- 2020 276 043
- US-B1- 11 141 304

## Beschreibung

Die vorliegende Erfindung betrifft eine Rückenorthese zum Stabilisieren eines Rückens eines Patienten.

Es gibt Erkrankungen, bei denen eine Krümmung einer Wirbelsäule eines Patienten auftritt. Eine derartige Krümmung kann auf eine angeborene Erkrankung zurückzuführen sein. Daneben gibt es auch Erkrankungen, die im Laufe des Lebens auftreten. Man denke in diesem Zusammenhang auch an übermäßige körperliche Belastungen, die eine Krümmung der Wirbelsäule bewirken. Als beispielhafte Krankheitsbilder seien stabile, osteoporotische Wirbelkörperfrakturen, Osteoporose, sekundäre Kyphosen, wie stabile Frakturen oder Tumore und muskuläre Insuffizienz genannt.

Um derartige Erkrankungen zu therapieren, sind neben chirurgischen Eingriffen auch konservative Methoden bekannt, die den Einsatz von stützenden Vorrichtungen wie Rückenorthesen vorsehen. Derartige Vorrichtungen stabilisieren den Rücken des Patienten und wirken wirbelsäulenaufrichtend.

Aus EP 0 917 864 B1 ist beispielsweise eine Rückenorthese zum Stabilisieren des Rückens eines Patienten bekannt. Diese Rückenorthese umfasst mehrere Komponenten, nämlich eine Rückenschiene, die am Rücken des Patienten anliegt. Ferner umfasst diese Gurte, um die Rückenschiene an dem Patienten zu befestigen und zusammen mit der Rückenschiene den Oberkörper und die Wirbelsäule des Patienten wieder aufzurichten.

Eine weitere Rückenorthese ist aus DE 20 2017 001 198 U1 bekannt. Diese Rückenorthese umfasst ein Zentralelement zur Stützung der Wirbelsäule eines Patienten und ein weiteres Stützelement zur Stützung eines Lenden- und/oder Hüftbereichs eines Patienten. Insofern ist das Stützelement an einem Rücken des Patienten unterhalb des Zentralelements angeordnet. An dem Zentralelement sind parallel zu einer Längsachse des Zentralelements verlaufende Langlöcher vorgesehen, um das Zentralelement mittels Schrauben mit dem Stützelement fest zu koppeln und in einem gelösten Zustand der Schrauben relativ zu dem Stützelement und entlang der Langlöcher zu verlagern.

DE 10 2006 043 845 A1, das als nächstliegender Stand der Technik angesehen wird, offenbart eine Brustwirbelsäulenorthese mit einem Schultergurtsystem, wobei ein Rückenteil rucksackartig getragen wird. Ferner sind an dem Rückenteil ein Tragriemensystem sowie ein auswechselbares Stützelement vorgesehen.

DE 10 2015 107 582 A1 offenbart eine Reklinationsorthese mit einem im getragenen Zustand der Orthese sich entlang des Rückens eines Patienten erstreckenden Stützelements. Die Reklinationsorthese weist ferner ein Verbindungselement auf, das das Stützelement mit einem Reklinationsbügel koppelt.

US 11 141 304 B1 offenbart eine weitere Orthese mit einem im getragenen Zustand der Orthese sich entlang des Rückens erstreckenden Elements.

Üblicherweise wird eine Rückenorthese von einem Arzt oder Therapeuten verordnet. Da die anatomischen Gegebenheiten der Patientenrücken und die pathologischen Ausprägungen von Rückenerkrankungen allerdings stark unterschiedlich ausfallen können, wird eine Rückenorthese häufig von einer geschulten Person aus einer Vielzahl an Produkten ausgewählt und ferner speziell an den Patienten angepasst. Dabei ist es von Vorteil, wenn die Rückenorthese einfach an die anatomischen Gegebenheiten anpassbar ist. Für einen Anbieter von Rückenorthesen ist es beispielsweise im Hinblick auf Kostenersparnisse von Vorteil, wenn eine Rückenorthese an möglichst viele anatomischen Gegebenheiten anpassbar ist und möglichst viele Krankheitsbilder abdecken kann.

Die aus dem Stand der Technik bekannten vorgefertigten Rückenorthesen sind allerdings in ihrer Anpassbarkeit an den Patienten beschränkt. Gleichzeitig geht eine höhere Anpassbarkeit beispielsweise an große Menschen häufig zu Lasten der Stabilisierungswirkung der Rückenorthese.

Es ist daher eine Aufgabe der vorliegenden Erfindung, eine Rückenorthese zum Stabilisieren eines Rückens eines Patienten bereitzustellen, die einfach und vielseitig an die Erfordernisse eines Patienten anpassbar ist.

Diese Aufgabe wird durch die Rückenorthese gemäß Patentanspruch 1 gelöst. Die abhängigen Ansprüche betreffen vorteilhafte Weiterbildungen der Erfindung.

Die Erfindung betrifft eine Rückenorthese zum Stabilisieren eines Rückens eines Patienten, umfassend
- ein erstes flächiges Stabilisierungselement zur Stützung des Rückens des Patienten;
- ein zweites flächiges Stabilisierungselement zur Stützung des Rückens des Patienten unterhalb des ersten flächigen Stabilisierungselements; und
- eine längliche Stabilisierungsschiene, die das erste und zweite flächige Stabilisierungselement miteinander koppelt und derart relativ zu dem ersten und/oder zweiten flächigen Stabilisierungselement geführt ist, dass eine geführte translatorische Verlagerung des ersten und des zweiten flächigen Stabilisierungselements zueinander in der Längsrichtung der Stabilisierungsschiene zur Anpassung der Rückenorthese an den Patienten möglich ist, gekennzeichnet durch zumindest ein Seitenteil zum zumindest teilweisen Umgreifen eines lateralen Körperbereichs des Patienten, wobei das Seitenteil mit dem ersten flächigen Stabilisierungselement und dem zweiten flächigen Stabilisierungselement gekoppelt oder koppelbar ist. Das erste und das zweite flächige Stabilisierungselement ermöglichen eine bedarfsgerechte Stützung bzw. Stabilisierung des Rückens. So kann über die Stabilisierungselemente gezielt Druck auf Bereiche des Rückens ausgeübt werden, an denen die

Stabilisierungselemente jeweils anliegen. Die längliche Stabilisierungsschiene koppelt die beiden Stabilisierungselemente in vorteilhafter Weise miteinander. Die translatorische Verlagerbarkeit erlaubt es, die beiden Stabilisierungselemente in unterschiedlichen Positionen entlang der Längsrichtung der Stabilisierungsschiene relativ zueinander auszurichten. So kann die Rückenorthese an bestimmte anatomische Gegebenheiten eines Patienten oder auch unterschiedliche Patienten, beispielsweise mit sich unterscheiden Körpergrößen, angepasst werden. So können die Stabilisierungselemente auch bei unterschiedlich großen Patienten an dazu vorgesehenen Körperregionen anliegen. Gleichzeitig bewirkt die Stabilisierungsschiene eine stabile Kopplung der beiden Stabilisierungselemente, was der stabilisierenden Wirkung der Rückenorthese zuträglich ist. Ferner kann die Stabilisierungsschiene abhängig von den Gegebenheiten bzw. Bedürfnissen des Patienten ausgewählt und an der Rückenorthese vorgesehen werden. Außerdem verleiht die Stabilisierungsschiene der Rückenorthese und insbesondere den Stabilisierungselementen zusätzliche Stabilität.

Es versteht sich, dass die Bestandteile der Rückenorthese als separate Bauteile ausgebildet sein können. So können beispielsweise das erste und das zweite flächige Stabilisierungselement und die längliche Stabilisierungsschiene separat, also als separate Bauteile, ausgebildet sein, die miteinander gekoppelt sind. Die Stabilisierungsschiene kann mit dem einen von dem ersten und zweiten flächigen Stabilisierungselement fest oder fixierbar gekoppelt sein und mit dem anderen von dem ersten und zweiten flächigen Stabilisierungselement geführt sein, dass die geführte translatorische Verlagerung möglich ist.

Gemäß einer Weiterbildung der Erfindung kann vorgesehen sein, dass die flächigen Stabilisierungselemente, insbesondere in Abhängigkeit der Verlagerung des ersten und/oder zweiten Stabilisierungselements relativ zu der Stabilisierungsschiene, relativ zueinander beabstandet angeordnet und/oder anordenbar sind.

Gemäß einem Aspekt der Erfindung kann vorgesehen sein, dass die längliche Stabilisierungsschiene im Wesentlichen mittig des ersten und/oder zweiten flächigen Stabilisierungselements angeordnet ist. Dies kann einen besonders einfachen Aufbau der Rückenorthese ermöglichen. Beispielsweise kann so auf weitere Stabilisierungsschienen verzichtet werden. Mit einer mittigen Anordnung kann dabei gemeint sein, dass die Stabilisierungsschiene in einem am Patienten angebrachten Zustand der Rückenorthese im Wesentliche entlang der Wirbelsäule des Patienten ausgerichtet ist, sich also die Längsrichtung der Stabilisierungsschiene im Wesentlichen entlang der Wirbelsäule erstreckt. Anders ausgedrückt kann die Stabilisierungsschiene in einem am Patienten angebrachten Zustand medial bzw. in der Saggitalebene liegend an dem Körper des Patienten angeordnet sein.

Gemäß einer Weiterbildung der Erfindung ist die Stabilisierungsschiene im Wesentlichen starr ausgebildet. Alternativ dazu kann die Stabilisierungsschiene in zumindest einer Körperrichtung, beispielsweise in einer Richtung orthogonal zur Längsrichtung der Stabilisierungsschiene, starr ausgebildet sein. Eine starre Ausbildung meint dabei eine vergleichsweise geringe und/oder lediglich mit erhöhtem Kraftaufwand zugelassene elastische Verformbarkeit der Stabilisierungsschiene. Ferner kann die Stabilisierungsschiene im Wesentlichen starr im Vergleich zu dem ersten und/oder zweiten flächigen Stabilisierungselement ausgebildet sein. So kann vorgesehen sein, dass bei Krafteinwirkung eine stärkere Verformung des ersten und/oder zweiten Stabilisierungselements verglichen mit der Stabilisierungsschiene auftritt. Die starre Ausbildung der Stabilisierungsschiene kann auf ihre geometrische Ausgestaltung und/oder die Materialwahl zurückzuführen sein. Ferner kann die Stabilisierungsschiene abhängig von den Gegebenheiten bzw. Bedürfnissen des Patienten ausgewählt und an der Rückenorthese vorgesehen werden. Insbesondere kann die Länge der Stabilisierungsschiene in der Längsrichtung in Abhängigkeit des Patienten vorgesehen sein.

Gemäß einer Weiterbildung der Erfindung kann vorgesehen sein, dass
- die Stabilisierungsschiene im Wesentlichen metallisches Material wie Stahl, Edelstahl, Titan, eine Metalllegierung usw. oder einen Verbundwerkstoff umfasst; und/oder
- das erste und/oder zweite flächige Stabilisierungselement im Wesentlichen Kunststoff, insbesondere thermoplastischen Kunststoff, Aluminium und/oder einen Kunststoff-Verbundwerkstoff umfasst.

Kunststoff kann dabei Polyethylen, Polypropylen, Polycarbonat Polyethylenterephthalat oder weitere Kunststoffe umfassen. Metallisches Material verleiht insbesondere eine hohe Biegefestigkeit und kann somit zur starren Ausbildung der Stabilisierungsschiene beitragen. Die im Zusammenhang mit den Stabilisierungselementen genannten Werkstoffe tragen in vorteilhafter Weise zu einem geringen Gewicht der Rückenorthese bei gleichzeitig hinreichender Stabilität bei.

Gemäß einer vorteilhaften Weiterbildung der Erfindung kann vorgesehen sein, dass die Stabilisierungsschiene fest mit einem von dem ersten oder zweiten flächigen Stabilisierungselement gekoppelt oder an diesem ausgebildet ist. In beiden Fällen ist die Stabilisierungsschiene dann nicht relativ zu dem ersten oder zweiten Stabilisierungselement verlagerbar. Dies ermöglicht zum einen eine stabile Anordnung der Stabilisierungsschiene relativ zu dem entsprechenden Stabilisierungselement. Andererseits kann damit ein einfacher Aufbau der Rückenorthese bewirkt werden. Dies erleichtert es beispielsweise, die Rückenorthese an einen Patienten anzupassen, denn es sind weniger relative Verlagerbarkeiten zugelassen.

Gemäß einem Aspekt der Erfindung kann vorgesehen sein, dass
- eine Breite der Stabilisierungsschiene ein Vielfaches einer Dicke der Stabilisierungsschiene beträgt; und/oder
- eine Länge der Stabilisierungsschiene in der Längsrichtung ein Vielfaches der Breite des Stabilisierungsschiene, beträgt; und/oder
- eine Breite des ersten und/oder zweiten flächigen Stabilisierungselements ein Vielfaches einer Breite der Stabilisierungsschiene beträgt.

So kann vorgesehen sein, dass die Stabilisierungsschiene im Vergleich zu zumindest einem der Stabilisierungselemente und zumindest in der Breitenrichtung der Stabilisierungsschiene klein ausfällt. Die Breitenrichtung ist dabei senkrecht zur Längsrichtung der Stabilisierungsschiene ausgerichtet und in einem am Patienten angebrachten Zustand der Rückenorthese in Richtung von medial nach lateral des Patienten ausgerichtet.

Gemäß einem Aspekt der Erfindung kann vorgesehen sein, dass die Stabilisierungsschiene das erste und zweite flächige Stabilisierungselement derart miteinander koppelt und derart relativ zu dem ersten und/oder zweiten flächigen Stabilisierungselement geführt ist, dass eine translatorische Verlagerung des ersten und des zweiten flächigen Stabilisierungselements zueinander in zumindest einer ersten zur Längsrichtung der Stabilisierungsschiene senkrecht ausgerichteten Richtung gesperrt und bevorzugt ferner in einer zweiten zur Längsrichtung der Stabilisierungsschiene senkrecht ausgerichteten Richtung gesperrt ist. Bevorzugt ist dabei vorgesehen, dass die erste und die zweite Richtung zueinander senkrecht ausgerichtet sind. Somit kann eine definierte Verlagerbarkeit der Stabilisierungselemente zueinander bereitgestellt werden. Dies erleichtert auch das Anpassen der Rückenorthese an den Patienten.

Gemäß einem weiteren Aspekt der Erfindung kann vorgesehen sein, dass die Stabilisierungsschiene ferner das erste und zweite flächige Stabilisierungselement derart miteinander koppelt und derart relativ zu dem ersten und/oder zweiten flächigen Stabilisierungselement geführt ist, dass eine rotatorische Verlagerbarkeit der flächigen Stabilisierungselemente zueinander gesperrt ist. Klarstellend sei angemerkt, dass eine elastische Verformbarkeit der flächigen Stabilisierungselemente bzw. der Stabilisierungsschiene davon ausgenommen ist. Die Kopplung bzw. Führung der Stabilisierungselemente über die Stabilisierungsschiene stellt jedoch keinen rotativen Bewegungsfreiheitsgrad bereit.

Gemäß einer Weiterbildung der Erfindung kann vorgesehen sein, dass das erste und/oder zweite flächige Stabilisierungselement von der Stabilisierungsschiene entkoppelbar ist. Vorzugsweise ist dabei vorgesehen, dass die Entkoppelbarkeit durch Verlagern relativ zu der Stabilisierungsschiene in deren Längsrichtung bereitgestellt wird, beispielsweise indem das erste und/oder das zweite flächige Stabilisierungselement in der Längsrichtung relativ zu der Stabilisierungsschiene verlagert wird. Insgesamt ermöglicht die Entkoppelbarkeit in vorteilhafter Weise, dass beispielsweise unterschiedliche Stabilisierungselemente miteinander koppelbar sind. Dies bedeutet, dass abhängig von einem Patienten und abhängig von der Beeinträchtigung des Rückens des Patienten zielgerichtet das erste oder das zweite Stabilisierungselement ausgesucht werden kann. So können mehrere erste und/oder mehrere zweite flächige Stabilisierungselemente und/oder Stabilisierungsschienen vorgesehen sein, die sich beispielsweise hinsichtlich ihrer Steifigkeit, ihrem Material, ihrer flächigen Erstreckung, der Kopplungspunkte des Gurtsystems zum Anlegen der Rückenorthese, ihrer Farbe usw. unterscheiden, wobei dann das passende Stabilisierungselement bzw. die passende Stabilisierungsschiene ausgewählt werden kann. So kann ein modularer Aufbau der Rückenorthese bereitgestellt werden.

Eine Weiterbildung der Erfindung sieht vor, dass die Rückenorthese eine Schienenbefestigungsanordnung zum Sperren der Verlagerbarkeit des ersten und zweiten flächigen Stabilisierungselements zueinander in der Längsrichtung der Stabilisierungsschiene umfasst. Dies ermöglicht es, insbesondere bei oder nach einem Anpassen der Rückenorthese an den Patienten die Rückenorthese in der Verlagerbarkeit in der Längsrichtung zu sperren, sodass während dem Tragen am Rücken des Patienten keine Verlagerung in der Längsrichtung zugelassen ist.

Gemäß einer Ausgestaltung der Erfindung kann vorgesehen sein, dass die Schienenbefestigungsanordnung dazu ausgebildet ist, in dem Sperrzustand eine Verlagerung der Stabilisierungsschiene relativ zu dem ersten oder zweiten flächigen Stabilisierungselement zu sperren.

Gemäß einem Aspekt der Erfindung kann vorgesehen sein, dass die Stabilisierungsschiene einen in der Längsrichtung der Stabilisierungsschiene ausgebildeten länglichen Schlitz umfasst, und die Schienenbefestigungsanordnung einen Feststellmechanismus, insbesondere eine Feststellschraube umfasst, die in dem länglichen Schlitz angeordnet ist und die zum Sperren die Stabilisierungsschiene relativ bzw. auf das erste oder zweite flächige Stabilisierungselement drückt.

Gemäß einer Weiterbildung der Erfindung kann vorgesehen sein, dass die Rückenorthese zumindest einen Vorspannmechanismus umfasst, der dazu eingerichtet ist, zwei zueinander verlagerbare Bestandteile der Rückenorthese relativ zueinander, insbesondere in Richtung aufeinander zu oder voneinander weg, mittels einer Spannkraft zu drängen. Durch den Vorspannmechanismus kann die Rückenorthese in einfacher Weise an die Gegebenheiten bzw. die Anatomie des Patienten angepasst werden. Dies ist insbesondere dann vorteilhaft, wenn Teile der Rückenorthese relativ zueinander zur Anpassung an den Patienten zu verlagern sind. So können die Teile dann in der gewünschten Lage beispielsweise mittels einer jeweiligen entsprechenden Befestigungsanordnung zumindest für die Dauer der Nutzung durch den Patienten relativ zueinander fixiert werden.

Gemäß der Erfindung umfasst die Rückenorthese zumindest ein Seitenteil zum zumindest teilweisen Umgreifen eines lateralen Körperbereichs des Patienten. So kann auch eine Stützung des lateralen Körperbereichs bereitgestellt werden. Dies kann der Krafteinbringung in dem lateralen Körperbereich oder der Abstützung der Rückenorthese gegenüber dem Körper des Patienten und zur besseren Krafteinbringung bzw. Stützung in einem anderen Körperbereich dienen.

Gemäß der Erfindung ist vorgesehen, dass das Seitenteil mit dem ersten und dem zweiten flächigen Stabilisierungselement gekoppelt oder koppelbar ist.

Gemäß einer Ausgestaltung der Erfindung umfasst die Rückenorthese ferner eine Führungsanordnung, die es ermöglicht, das zumindest eine Seitenteil relativ zu dem ersten und/oder zweiten flächigen Stabilisierungselement beispielsweise in einer Seitenrichtung zu verlagern.

Zusätzlich oder alternativ kann vorgesehen sein, dass die Rückenorthese eine Führungsanordnung oder die vorangehend genannte Führungsanordnung umfasst, die es ermöglicht, die flächigen Stabilisierungselemente relativ zueinander zu verlagern.

Gemäß einem Aspekt der Erfindung ist der Vorspannmechanismus dazu eingerichtet, das Seitenteil in der Seitenrichtung, insbesondere in medialer Richtung, relativ zu dem ersten und/oder zweiten flächigen Stabilisierungselement mittels der Spannkraft zu drängen.

Gemäß einer Weiterbildung der Erfindung ist die Führungsanordnung ferner dazu ausgebildet, das Seitenteil in der Längsrichtung, insbesondere einer Höhenrichtung, relativ zu dem ersten und/oder zweiten flächigen Stabilisierungselement zu verlagern.

Gemäß einem Aspekt der Erfindung umfasst die Führungsanordnung einen Sperrmechanismus zum Sperren der Verlagerbarkeit des Seitenteils relativ zu dem ersten und/oder zweiten flächigen Stabilisierungselement und/oder zum Sperren der Verlagerbarkeit der Stabilisierungselemente relativ zueinander.

Gemäß einem Aspekt der Erfindung umfasst die Rückenorthese zwei Seitenteile zum zumindest teilweisen Umgreifen eines lateralen Körperbereichs des Patienten, wobei das erste Seitenteil dazu eingerichtet ist, einen linken lateralen Körperbereich zu umgreifen, und das zweite Seitenteil dazu eingerichtet ist, einen rechten lateralen Körperbereich zu umgreifen. Merkmale, die im Zusammenhang mit einem Seitenteil erläutert wurden, können jeweils auch an den beiden Seitenteilen vorgesehen sein.

Gemäß einer Ausgestaltung der Erfindung kann vorgesehen sein, dass der Betrag der Spannkraft des Vorspannmechanismus einstellbar ist. Man denke an eine einstellbare Spannkraft einer Feder oder eine Austauschbarkeit der Feder.

Gemäß einer weiteren Ausgestaltung der Erfindung kann vorgesehen sein, dass der Vorspannmechanismus einen elastischen Vorspanngurt umfasst, der bevorzugt elastisches, besonders bevorzugt gummielastisches, Material umfasst.

Gemäß einer Weiterbildung der Erfindung kann vorgesehen sein, dass das Seitenteil insbesondere an dessen dem ersten und/oder zweiten flächigen Stabilisierungselement abgewandten Ende flexible Bereiche umfasst, die eine gesteigerte elastische Verformbarkeit des Seitenteils bewirken. Bevorzugt ist dabei vorgesehen, dass die flexiblen Bereiche Materialaussparungen umfassen. Besonders bevorzugt sind die Materialaussparungen ausgehend von einem Randbereich des Seitenteils in dieses hinein erstreckend und/oder als längliche Schlitze ausgebildet. Die flexiblen Bereiche steigern den Tragekomfort für einen Patienten und dessen Bewegungsfreiheit. Zusätzlich oder alternativ können die flexiblen Bereiche auch an anderen Komponenten der Rückenorthese ausgebildet sein, beispielsweise an dem ersten und/oder zweiten Stabilisierungselement. Dann sind die flexiblen Bereiche vorzugsweise an zumindest einem Randbereich, insbesondere zumindest einem lateralen Randbereich, des ersten/und oder zweiten Stabilisierungselements ausgebildet.

Gemäß einer Weiterbildung der Erfindung umfasst die Rückenorthese ferner eine insbesondere zweiteilige Hüftgurtanordnung, die an dem Seitenteil und/oder einem der Seitenteile und/oder dem ersten und/oder zweiten flächigen Stabilisierungselement angebracht oder anbringbar ist, und dazu eingerichtet ist, einen hüftnahen Bereich des Patienten zu umgreifen.

Gemäß einer weiteren Weiterbildung der Erfindung umfasst die Hüftgurtanordnung einen betätigbaren Spannmechanismus, insbesondere einen Seilzugmechanismus, der dazu eingerichtet ist, die beiden Teile der Hüftgurtanordnung bei Betätigung zueinander, bevorzugt in Umfangsrichtung des Patienten, zu verspannen. Dies kann es einem Patienten erleichtern, die Rückenorthese zum Tragen anzulegen bzw. diese nach Gebrauch abzulegen. Ferner erleichtert es der Spannmechanismus, die Hüftgurtanordnung an den Patienten bzw. nach dessen Bedürfnissen anzupassen.

Gemäß einer Weiterbildung der Erfindung kann vorgesehen sein, dass die Rückenorthese ferner eine Schultergurtanordnung mit wenigstens zwei Schultergurtzügen umfasst. Bevorzugt ist dabei vorgesehen, dass ein jeweiliger Schultergurtzug mit dessen jeweiligen einen Bereich an das erste flächige Stabilisierungselement und mit dessen jeweiligen anderen Bereich an dem zweiten flächigen Stabilisierungselement koppelbar oder gekoppelt ist. Außerdem kann vorgesehen sein, dass jeder Schultergurtzug bei Anbringung am Patienten insbesondere von dem ersten flächigen Stabilisierungselement über eine Schulter des Patienten geführt oder führbar ist. Die Schultergurtanordnung ermöglicht somit ein angenehmes Tragen der Rückenorthese am Patienten, sowie ein einfaches An-und Ablegen der Rückenorthese.

Gemäß einer Weiterbildung der Erfindung kann vorgesehen sein, dass die Schultergurtanordnung ferner eine erste Kopplungsanordnung umfasst, die dazu eingerichtet ist, die beiden Schultergurtzüge bei Anbringung am Patienten insbesondere bauchseits zu koppeln. Die Kopplungsanordnung kann einen Klettverschluss, verrastbare Schnallen oder dergleichen umfassen.

Gemäß einer Weiterbildung der Erfindung kann vorgesehen sein, dass zumindest einer der Schultergurtzüge zweiteilig ausgebildet ist und eine zweite Kopplungsanordnung umfasst, die dazu eingerichtet ist, die beiden Teile des Schultergurts miteinander zu koppeln.

Gemäß einer Weiterbildung der Erfindung kann vorgesehen sein, dass die Rückenorthese, insbesondere das erste Stabilisierungselement, zumindest ein Begrenzungsmittel umfasst, das dazu eingerichtet ist, einen Winkelbereich vorzugeben, innerhalb dessen ein jeweiliges Ende des jeweiligen Schultergurtzugs, das mit der Rückenorthese gekoppelt ist, relativ zu der Rückenorthese verlagerbar bzw. verdrehbar ist. Das Begrenzungsmittel kann zumindest eine, bevorzugt zwei, Erhebungen umfassen. Die zumindest eine Erhebung kann die Funktion eines Anschlags aufweisen, der die Drehung des Endes des Schultergurtzugs begrenzt. Bevorzugt sind die Erhebungen auf einer imaginären Kreisbahn um ein jeweiliges Durchgangsloch angeordnet, wobei der jeweilige Schultergurtzug innerhalb des Winkelbereichs um eine Achse des Durchgangsloch relativ zu dem ersten Stabilisierungselement verlagerbar ist. Vorzugsweise umfasst das Ende des jeweiligen Schultergurtzugs eine Umlenklasche, die den Schultergurtzug mit dem ersten Stabilisierungselement koppelt. Die Umlenklasche ist wiederum bevorzugt an dem Durchgangsloch angebracht und um eine Längsachse des Durchgansloch drehbar, wobei die Drehbarkeit durch das Begrenzungsmittel auf den Winkelbereich beschränkt ist.

Ein weiterer Aspekt der Offenbarung betrifft eine Rückenorthese zum Stabilisieren eines Rückens eines Patienten, umfassend ein flächiges Stabilisierungselement zur Stützung des Rückens des Patienten. Alle vorangehend erläuterten Ausgestaltungsformen bzw. Merkmale können an dieser Rückenorthese vorgesehen bzw. realisiert sein. Merkmale, die an oder im Zusammenhang mit einem der vorangehend aufgeführten Stabilisierungselemente erläutert wurden, können an dem oder in Zusammenhang mit dem vorliegenden Stabilisierungselement vorgesehen sein.

Insbesondere kann vorgesehen sein, dass die vorangehend genannte Rückenorthese den Vorspannmechanismus umfasst, der dazu eingerichtet ist, zwei zueinander verlagerbare Bestandteile der Rückenorthese relativ zueinander, insbesondere in Richtung aufeinander, mittels einer Spannkraft zu drängen. Dazu kann die Rückenorthese zumindest ein Seitenteil zum zumindest teilweisen Umgreifen eines lateralen Körperbereichs des Patienten umfassen. Der Vorspannmechanismus kann dazu eingerichtet sein, das Seitenteil relativ zu dem flächigen Stabilisierungselement zu drängen.

Gemäß einer Ausführungsform kann die Rückenorthese flexible Bereiche umfassen, die eine gesteigerte elastische Verformbarkeit insbesondere des Seitenteils bewirken. Bevorzugt umfassen die flexiblen Bereiche Materialaussparungen, die ausgehend von einem Randbereich des Seitenteils in dieses hinein erstreckend ausgebildet sind. Dazu kann die Rückenorthese zumindest ein Seitenteil zum zumindest teilweisen Umgreifen eines lateralen Körperbereichs des Patienten umfassen. Die flexiblen Bereiche können an dem Seitenteil, insbesondere an dessen dem flächigen Stabilisierungselement abgewandten Ende, ausgebildet sein.

Gemäß einer weiteren Ausführungsform kann die Rückenorthese eine insbesondere zweiteilige Hüftgurtanordnung umfassen, die an dem Seitenteil und/oder zumindest einem der Seitenteile und/oder dem flächigen Stabilisierungselement angebracht oder anbringbar ist, und dazu eingerichtet ist, einen hüftnahen Bereich des Patienten zu umgreifen. Ferner kann vorgesehen sein, dass die Hüftgurtanordnung einen betätigbaren Spannmechanismus, insbesondere einen Seilzugmechanismus, umfasst, der dazu eingerichtet ist, die beiden Teile der Hüftgurtanordnung bei Betätigung zueinander, bevorzugt in Umfangsrichtung des Patienten, zu verspannen. An dem Stabilisierungselement kann zumindest ein Führungsschlitz ausgebildet sein, der dazu vorgesehen ist, die Hüftgurtanordnung, insbesondere ein Hüftgurtteil der Hüftgurtanordnung, relativ zu dem Stabilisierungselement zu führen.

Gemäß einer weiteren Ausführungsform kann die Rückenorthese eine Schultergurtanordnung mit wenigstens zwei Schultergurtzügen umfassen. Es kann dann vorgesehen sein, dass ein jeweiliger Schultergurtzug zwei Bereiche des Stabilisierungselements, das Stabilisierungselement mit der Hüftgurtanordnung oder einem der Seitenteile oder beliebige andere Teile der Rückenorthese miteinander koppelt oder mit diesen koppelbar ist. So kann vorgesehen sein, dass ein jeweiliger Schultergurtzug mit dessen jeweiligen einen Ende an das flächige Stabilisierungselement oder die Hüftgurtanordnung koppelbar oder gekoppelt ist und mit dessen jeweiligen anderen Ende an das flächige Stabilisierungselement koppelbar oder gekoppelt ist.

Allgemein können einzelne Merkmale, die im Zusammenhang mit einer der vorangehend beschriebenen Rückenorthesen oder einem Bauteil derselben erläutert wurden, isoliert und als eigenständige Lehre betrachtet werden. Ferner können diese Merkmale auf eine der anderen Rückenorthesen oder Bauteile derselben übertragen werden.

Die Erfindung wird nachfolgend anhand der beigefügten Zeichnungen weiter erläutert. Es stellen dar:
- Fig. 1: eine schematische Rückansicht einer Rückenorthese gemäß einem Ausführungsbeispiel;
- Fig. 2: eine schematische Vorderansicht der Rückenorthese gemäß dem Ausführungsbeispiel;
- Fig. 3: eine schematische vereinfachte Rückansicht der Rückenorthese gemäß dem Ausführungsbeispiel;
- Fig. 4: eine schematische vereinfachte Vorderansicht der Rückenorthese gemäß dem Ausführungsbeispiel;
- Fig. 5a: eine schematische vereinfachte Seitenansicht der Rückenorthese gemäß dem Ausführungsbeispiel;
- Fig. 5b: eine schematische vereinfachte Untersicht des ersten Stabilisierungselements gemäß dem Ausführungsbeispiel;
- Fig. 5c: eine schematische vereinfachte isometrische Ansicht der Stabilisierungsschiene gemäß dem Ausführungsbeispiel;
- Fig. 6: eine schematische vereinfachte Rückansicht der Rückenorthese gemäß einem weiteren Ausführungsbeispiel;
- Fig. 7: eine schematische vereinfachte isometrische Ansicht der Rückenorthese gemäß dem weiteren Ausführungsbeispiel;
- Fig. 8: eine schematische vereinfachte isometrische Ansicht der Rückenorthese gemäß einem weiteren Ausführungsbeispiel; und
- Fig. 9: eine schematische vereinfachte Rückansicht der Rückenorthese gemäß dem weiteren Ausführungsbeispiel gemäß Figur 8.

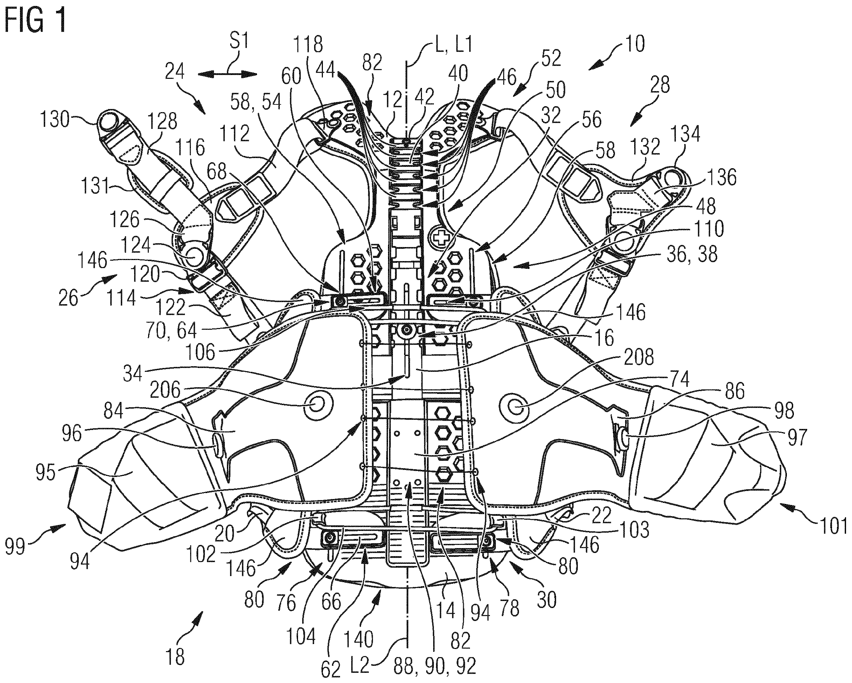

Figur 1 betrifft eine schematische Darstellung einer Rückenorthese 10 zum Stabilisieren eines Rückens eines Patienten in einer Rückansicht. Die Rückenorthese 10 umfasst ein erstes flächiges Stabilisierungselement 12 und ein zweites flächiges Stabilisierungselement 14. Eine längliche Stabilisierungsschiene 16 koppelt das erste und das zweite flächige Stabilisierungselement 12, 14 miteinander. Ferner ist das erste flächige Stabilisierungselement 12 derart relativ zu der länglichen Stabilisierungsschiene 16 geführt, dass eine geführte translatorische Verlagerung des ersten und des zweiten flächigen Stabilisierungselement 12, 14 zueinander in einer Längsrichtung L der Stabilisierungsschiene 16 zur Anpassung der Rückenorthese an den Patienten möglich ist. Die Rückenorthese 10 umfasst ferner eine Hüftgurtanordnung 18, sowie ein erstes Seitenteil 20 und ein zweites Seitenteil 22. Die Seitenteile 20, 22 sind jeweils zum zumindest teilweisen Umgreifen eines lateralen Körperbereichs des Patienten vorgesehen. Ferner umfasst die Rückenorthese 10 eine Schultergurtanordnung 24 umfassend einen ersten Schultergurtzug 26 und einen zweiten Schultergurtzug 28, wobei die beiden Schultergurtzüge 26, 28 jeweils zum einen mit dem ersten flächigen Stabilisierungselement 12 und dem zweiten flächigen Stabilisierungselement 14 bzw. einem daran angebrachten Polsterelement 140 gekoppelt sind.

In einem mittigen Bereich des ersten Stabilisierungselements 12 ist eine Stabilisierungsführung 32 ausgebildet, in der die Stabilisierungsschiene 16 aufgenommen und relativ zu dem ersten Stabilisierungselement 12 geführt ist. Die Stabilisierungsführung 32 bildet somit eine Aufnahmeöffnung des ersten Stabilisierungselements 12 zur Aufnahme der Stabilisierungsschiene 16 und erstreckt sich in Richtung einer Längsachse L1 des ersten Stabilisierungselements 12, die in dem mit der Stabilisierungsschiene 16 gekoppelten Zustand mit der Längsrichtung L zusammenfällt. In der Stabilisierungsschiene 16 ist ein Längsschlitz 34 ausgebildet, der sich in der Längsrichtung L erstreckt. Ferner ist eine Schienenbefestigungsanordnung 36 vorgesehen umfassend eine Feststellschraube 38, die mit einem Schraubenkopf an einer Rückseite der Stabilisierungsschiene 16 anliegt und mit ihrem Schraubenschaft durch den Längsschnitt 34 ragt und mit dem ersten flächigen Stabilisierungselement 12 oder einem daran anliegenden oder in diesem ausgebildetem oder ausgebildetem Gewindeelement verschraubt ist. Die Feststellschraube 38 umfasst zur verbesserten Krafteinleitung einen vergrößerten Kopf bzw. ordnet eine Unterlegscheibe zwischen ihr und der Stabilisierungsschiene 16 an. In einem festen Zustand der Schienenbefestigungsanordnung 36 bewirkt die Feststellschraube 38 in Zusammenspiel mit dem Gewindeelement, dass die Stabilisierungsschiene 16 und das erste flächige Stabilisierungselement 12 aufeinander gepresst werden, sodass eine relative Verlagerung der beiden zueinander gesperrt ist. In einem gelösten Zustand der Schienenbefestigungsanordnung 36 werden die Stabilisierungsschiene 16 und das erste flächige Stabilisierungselement 12 jedoch nicht oder nur geringfügig aufeinandergepresst und es ist somit eine relative Verlagerung der Stabilisierungsschiene 16 relativ zu der Stabilisierungsführung 32 möglich.

An dem ersten Stabilisierungselement 12 erstreckt sich die Stabilisierungsführung 32 über die gesamte Länge des Stabilisierungselements 12 entlang der Längsachse L1. Während ein dem zweiten Stabilisierungselement 14 zugewanderter Teil der Stabilisierungsführung 32 dazu dient, die Stabilisierungsschiene 16 aufzunehmen, ist ein dem zweiten Stabilisierungselement 14 abgewanderter Teil der Stabilisierungsführung 32 - ein oberer Teil des ersten Stabilisierungselements 12 - dazu vorgesehen, einen Stabilisierungskörper 40 aufzunehmen. Der Stabilisierungskörper 40 ist im Vergleich zu dem ersten Stabilisierungselement 12 im Wesentlichen starr ausgebildet und dient dazu, einen oberen Bereich des ersten Stabilisierungselements 12 zusätzlich zu stabilisieren. Der Stabilisierungskörper 40 ist an dem ersten Stabilisierungselement 12 fest angebracht, indem eine Schraube 42 den Stabilisierungskörper 40 an dem ersten Stabilisierungselement 12 fixiert. Ferner umfasst der obere Teil der Stabilisierungsführung 32 eine Vielzahl an Befestigungselementen 44, die beanstandet zueinander angeordnet sind und den Stabilisierungskörper 40 an dessen Rückseite umgreifen, sodass dieses spielfrei in der Stabilisierungsführung 32 angeordnet ist. Der Stabilisierungskörper 40 umfasst im Wesentlichen eine Grundform basierend auf einem Rechteck, das eine vorgegebene Materialdicke aufweist und zueinander beanstandete seitliche Materialaussparungen 46 bzw. Verjüngungen umfasst, die bei dem Stabilisierungskörper 40 gemäß der vorliegenden Anordnung im Bereich zwischen den Befestigungselementen 44 zu einer gesteigerten Biegbarkeit beitragen. Insofern dienen die Materialaussparungen 46 dazu, den ansonsten starr ausgebildeten Stabilisierungskörper 40 stellenweise biegbarer auszugestalten. Diese Biegbarkeit ermöglicht es, an dem Stabilisierungskörper 40 vorab oder im bereits in der Stabilisierungsführung 32 angeordneten Zustand eine Krümmung mittels plastischer Verformung desselben auszubilden. Aufgrund der höheren Steifigkeit des Stabilisierungskörpers 40 gegenüber dem ersten Stabilisierungselement 12 wird auch der mittlere Bereich 50 des ersten Stabilisierungselements 12 entsprechend gekrümmt ausgebildet sein. Dies ermöglicht es, die Rückenorthese 12, insbesondere das erste Stabilisierungselement 12, an einen Patienten einfach anzupassen.

Das erste Stabilisierungselement 12 umfasst einen unteren Bereich 48, einen mittleren Bereich 50 und einen oberen Bereich 52. Der untere Bereich 48 ist gegenüber dem mittleren Bereich 50 breiter ausgebildet und der obere Bereich 52 ist wiederum gegenüber dem unteren Bereich 48 und dem mittleren Bereich 50 breiter ausgebildet. Der obere Bereich 52 bildet zusammen mit dem mittleren Bereich 50 eine Y-förmige Gestalt. Der obere Bereich 52 dient dazu, in einem am Patienten angebrachten Zustand an einem Schulterbereich des Rückens des Patienten anzuliegen. Der mittlere Bereich 50 und der untere Bereich 48 dienen dazu, in einem am Patienten angebrachten Zustand im Bereich der Brustwirbelsäule des Rückens anzuliegen.

Das erste Stabilisierungselement 12 umfasst außerdem beiderseits der Stabilisierungsführung 32 und parallel zu der Längsachse L1 ausgebildete Führungsschlitze 54, 56. Diese bilden einen Teil einer jeweiligen Führungsanordnung 58, die es ermöglicht, das Seitenteil entlang der Längsachse L1 relativ zu dem ersten Stabilisierungselement 12 zu verlagern. Insofern führt eine Verlagerung des ersten Stabilisierungselements 12 relativ zu der Stabilisierungsschiene 16 auch zu einer Verlagerung des ersten Stabilisierungselements 12 relativ zu dem jeweiligen Seitenteil 20, 22.

Die Führungsanordnung 58 wird im Folgenden mit Bezug auf das erste Seitenteil 20 und das erste Stabilisierungselement 12 erläutert, wobei die Führungsanordnung 58 bezüglich des zweiten Seitenteils 22 relativ zu dem ersten Stabilisierungselement 12 sowie die jeweilige Führungsanordnung 80 bezüglich der Seitenteile 20, 22 relativ zu dem zweiten Stabilisierungselement 14 analog ausgebildet ist. Das erste Seitenteil 20 umfasst einen oberen Führungsfortsatz 60 und einen unteren Führungsfortsatz 62. In den jeweiligen Führungsfortsätzen 60, 62 sind jeweilige Führungsschlitze 64, 66 ausgebildet, die im an die Stabilisierungselemente 12, 14 angebrachten Zustand im Wesentlichen senkrecht zur Längsachse L1 angeordnet sind (abhängig von einer Neigung des Seitenteils 20 relativ zu den Stabilisierungselementen 12, 14) und somit eine Verlagerbarkeit des ersten Seitenteils 20 quer zur Längsachse L1 und damit in einer Seitenrichtung S1 des Patienten (medial - lateral) relativ zu den Stabilisierungselementen 12, 14 bereitstellt. Ferner ermöglicht die Verlagerbarkeit aufgrund des Zusammenspiels der beiden Führungsanordnungen 58, 80 eine Verkippung des Seitenteils 20 relativ zu den Stabilisierungselementen 12, 14. Auch dies verbessert die Anpassbarkeit der Rückenorthese an verschiedene Patienten. Ferner sind die Führungsfortsätze 60, 62 in deren Form an die Kontur des ersten und zweiten Stabilisierungselements 12, 14 im Bereich der Führungsschlitze 54, 56 angepasst. Die Führungsanordnung 58 umfasst eine Feststellschraube 68 und einen Führungskörper 70, der in dem Führungsschlitz 64, genauer gesagt in einer um den Führungsschlitz 64 ausgebildeten Führungsvertiefung 72 des Führungsfortsatzes 60 geführt ist. Die Feststellschraube 68 befestigt den Führungskörper 70 in der Führungsvertiefung 72 und ragt durch den Führungsschlitz 64 und den Führungsschlitz 56 und ist mit einem nicht dargestellten weiteren Führungskörper innenseitig des ersten Stabilisierungselements 12 verschraubt, der analog zu dem Führungskörper 70 in einer Führungsvertiefung des ersten Stabilisierungselements 12 im Bereich des Führungsschlitzes 54 geführt ist. Insofern bilden die Feststellschraube 68 und der weitere Führungskörper durch das einstellbare Klemmen des Seitenteils 20 und des ersten Stabilisierungselements 12 einen Sperrmechanismus. In einem gelockerten Zustand der Feststellschraube 68 ermöglicht die Führungsanordnung 58 eine Verlagerung des ersten Stabilisierungselement 12 und des ersten Seitenteils 20 relativ zueinander in Richtung der Längsachse L1 und in der Seitenrichtung S1, wohingegen in einem befestigten Zustand der Feststellschraube 68 jeweilige Verlagerungen gesperrt sind.

Das zweite Stabilisierungselement 14 weist in dessen mittleren Bereich eine Aufnahmetasche 74 auf, deren Längsachse L2 in einem mit der Stabilisierungsschiene 12 gekoppeltem Zustand mit der Längsrichtung L zusammenfällt. Die Aufnahmetasche 74 ist in einem oberen Bereich geöffnet ausgebildet. Ferner erstreckt sich ein liegender Hohlraum der Aufnahmetasche 74 entlang der Längsachse L2 bis in einen unteren Bereich des zweiten Stabilisierungselements 14 und ist nach unten und zu den Seiten hin geschlossen ausgebildet. Die Aufnahmetasche 74 ist dazu ausgebildet, die Stabilisierungsschiene 16 aufzunehmen. So wird die Stabilisierungsschiene 16 zur Montage der Rückenorthese 10 in die Aufnahmetasche 74 eingeführt, wobei Verbindungsmittel wie beispielsweise Klebstoff, Schraubenverbindungen, eine Presspassung, Umspritzung mit Kunststoff oder dergleichen vorgesehen sein können, um die Stabilisierungsschiene 12 fest mit dem zweiten Stabilisierungselement 14 zu koppeln. Die Aufnahmetasche 74 ist ausgehend von einem oberen Ende des zweiten Stabilisierungselements 14, also einem dem ersten Stabilisierungselement 12 zugewandten Bereich, entlang der Längsachse L2 fast, jedoch nicht vollständig, bis zu einem unteren Ende des zweiten Stabilisierungselements 14 ausgebildet. Die Anordnung der Stabilisierungsschiene 16 innerhalb der Aufnahmetasche 74 dient dazu, das zweite Stabilisierungselement 14 zusätzlich zu stabilisieren.

Analog zu den Führungsschlitzen 54, 56 des ersten Stabilisierungselements 12 sind auch an dem zweiten Stabilisierungselement 14 entsprechende Führungsschlitze 76, 78 ausgebildet, die beiderseits der Aufnahmetasche 74 und parallel zu der Längsachse L2 ausgebildete sind. Diese bilden einen Teil der jeweiligen Führungsanordnung 80, die es ermöglicht, das Seitenteil 20 entlang der Längsachse L2 relativ zu dem zweiten Stabilisierungselement 14 zu verlagern. Insofern führt eine Verlagerung des zweiten Stabilisierungselements 14 relativ zu dem ersten Stabilisierungselement 12 auch zu einer Verlagerung des zweiten Stabilisierungselements 14 relativ zu dem jeweiligen Seitenteil 20, 22.

Die jeweiligen Stabilisierungselemente 12, 14 umfassen jeweilige wabenförmige hexagonale Materialaussparungen 82, die in hinreichendem Abstand voneinander mit ausreichend starken Zwischenstreben verteilt sind und die dazu dienen, Gewicht an der Rückenorthese 10 einzusparen. Ferner dienen die Materialaussparungen 82 dazu, das ästhetische Erscheinungsbild der Rückenorthese 10 zu verbessern. Die Materialaussparung in 82 können als vollständige Durchbrüche in den jeweiligen Stabilisierungselementen 12, 14 oder nur als lokale Vertiefungen ausgebildet sein. Sie können in sämtlichen Stabilisierungselementen der erfindungsgemäßen Rückenorthese verteilt angeordnet sein, wie im folgenden noch erwähnt sein wird.

Die Hüftgurtanordnung 18 umfasst ein erstes Hüftgurtteil 84 und ein zweites Hüftgurtteil 86. Das erste Hüftgurtteil 84 liegt an dem ersten Seitenteil 20 an und umfasst auf dessen dem ersten Seitenteil 20 zugewandten Seite eine erste Lasche, die mit einer weiteren an dem ersten Seitenteil 20 angebrachten Lasche gekoppelt ist. Die beiden Laschen sind derart ausgebildet, dass eine Verlagerung des ersten Hüftgurtteils 84 in der Seitenrichtung S1 relativ zu dem ersten Seitenteil 20 in einem vorgegebenen Bereich zugelassen ist, während eine Verlagerung des ersten Hüftgurtteils 84 in einer Höhenrichtung, als beispielsweise in Längsrichtung L oder in Richtung einer der Längsachsen L1, L2 nur in einem im Vergleich dazu kleinen oder keinem Bereich zugelassen ist.

Die Hüftgurtanordnung 18 umfasst einen betätigbaren Spannmechanismus 88 umfassend eine Zugseilanordnung 90, wobei zumindest ein Zugseil 92 die beiden Hüftgurtteile 84, 86 miteinander koppelt und durch jeweilige Zugseilöffnungen 94 der Hüftgurtteile 84, 86 geführt ist. Der Spannmechanismus 88 ist dazu eingerichtet, derart betätigt zu werden, dass das Zugseil 92 im Bereich zwischen den Hüftgurtteilen 84, 86 verkürzt wird. Dazu sind an den Hüftgurtteilen 84, 86 jeweilige Drehknöpfe 96, 98 vorgesehen, die bei deren Drehung dazu ausgebildet sind, das Zugseil 92 innerhalb der jeweiligen Hüftgurtteile 84, 86 aufzurollen. Das Zugseil 92 kann gemäß dem Prinzip eines Flaschenzugs in dem jeweiligen Hüftgurtteil 84, 86 geführt sein. Die Drehknöpfe 96, 98 sind ferner dazu ausgebildet, in Richtung aus dem Hüftgurtteil 84, 86 um einen vorgegebenen Betrag herausgezogen zu werden, wobei dann der Spannmechanismus 88 das Zugseil 92 freigibt, sodass die beiden Hüftgurtteile 84, 86 erneut voneinander in der Seitenrichtung S1 entfernt werden können. Bei erneuter Verlagerung der Drehknöpfe 96, 98 in Richtung des jeweiligen Hüftgurtteils 84, 86 kann das Zugseil 92 mittels Betätigung der Drehknöpfe 96, 98 aufgerollt werden.

An dem ersten Hüftgurtteil 84 ist eine erste Greiflasche 95 und an dem zweiten Hüftgurtteil 86 ist eine zweite Greiflasche 97 ausgebildet. Die beiden Greiflasche 95, 97 dienen dazu, von einem Patienten während des Anlegens der Hüftgurtanordnung 18 ergriffen zu werden, um die Hüftgurtanordnung zu spannen. Darüber hinaus umfassen die beiden Hüftgurtteile 84, 86 jeweilige Klettverschlussanordnungen 99, 101 an dem jeweiligen, dem Spannmechanismus 88 abgewandten Ende der Hüftgurtteile 84, 86. Die Klettverschlussanordnungen 99, 101 sind dazu ausgebildet, mit dem jeweiligen anderen Hüftgurtteil 84, 86 gekoppelt zu werden, um die Hüftgurtanordnung 18 im vorderen Bauchbereich zu schließen, sodass der Bauchbereich des Patienten durch die Hüftgurtanordnung 18 umgriffen wird. Vorliegend ist die Klettverschlussanordnung 99 außenliegend - also in einem am Patienten angeordneten Zustand der Hüftgurtanordnung 18 auf einer vom Patienten weg gerichteten - Seite des Hüftgurtteils 84, während die Klettverschlussanordnung 101 auf einer innenliegenden - also in einem am Patienten angeordneten Zustand der Hüftgurtanordnung 18 auf einer zum Patienten hin gerichteten - Seite des Hüftgurtteils 86 ausgebildet ist. Insofern ist das erste Hüftgurtteil 84 dazu vorgesehen, an einer Bauchseite des Patienten angeordnet zu sein und von dem zweiten Hüftgurtteil 86 zumindest teilweise umgriffen bzw. umspannt zu sein.

Die Rückenorthese 10 umfasst ferner einen ersten Vorspannmechanismus 100, der dazu eingerichtet ist, die beiden Seitenteile 20, 22 relativ zueinander, nämlich in Richtung aufeinander, mittels einer Spannkraft zu drängen. Dazu ist an den Seitenteilen 20, 22 jeweilige Spannhaken 102, 103 in einem unteren Bereich der Seitenteile 20, 22 ausgebildet. Der erste Vorspannmechanismus 100 umfasst ferner einen ersten elastischen Vorspanngurt 104, der um den jeweiligen Spannhaken 102, 103 geführt ist und somit die Spannkraft auf die Spannhaken 102, 103 überträgt. Abhängig von beispielsweise der Länge, dem Material, der Dicke und der Elastizität des ersten Vorspanngurts 104 kann der Betrag der Spannkraft bedarfsabhängig angepasst werden. Analog zu dem ersten Vorspannmechanismus 100 ist ein zweiter Vorspannmechanismus 106 vorgesehen, wobei dazu in einem oberen Bereich der jeweiligen Seitenteile 20, 22, der vorliegend von der Hüftgurtanordnung 18 verdeckt ist, ebenfalls Spannhaken 108, 109 ausgebildet sind und ein zweiter elastischer Vorspanngurt 110 um die zweiten Spannhaken 106 geführt ist. Die beiden Vorspannmechanismen 100, 106 dienen dazu, in einem gelockerten Zustand der jeweiligen Feststellschraube 68 der jeweiligen Führungsanordnungen 58, 80 die beiden Seitenteile 20, 22 relativ zueinander und relativ zu dem ersten und dem zweiten Stabilisierungselement 12, 14 zu verlagern. Dies ist von besonderem Vorteil, wenn die Rückenorthese 10 an einen Patienten angepasst wird. Denn dann kann die jeweilige bzw. alle Feststellschrauben 68 gelockert und die Rückenorthese 10 einem Patienten angelegt werden. Die Vorspannmechanismen 100, 106 dienen dazu, die Seitenteile 20, 22 relativ zueinander und relativ zu den beiden Stabilisierungselementen 12, 14 entsprechend der Körperkontur des Patienten auszurichten. Dies erleichtert die Anpassung der Rückenorthese 10 an einen Patienten. Gegebenenfalls ist es notwendig, die relative Lage zusätzlich manuell zu korrigieren. Anschließend kann die jeweilige Feststellschraube 68 angezogen werden, um die Führungsanordnungen 58, 80 zu fixieren bzw. zu sperren. Üblicherweise sind die Führungsanordnungen 58, 80 in einem Zustand der Verwendung der Rückenorthese 10 zur Stabilisierung des Rückens eines Patienten, also während der Therapie, fixiert bzw. gesperrt.

Der erste Schultergurtzug 26 umfasst ein oberes Schultergurtteil 112 und ein unteres Schultergurtteil 114, die über ein Brustpolsterelement 116 miteinander gekoppelt sind. Das obere Schultergurtteil 112 ist mit dessen einen Ende an dem Brustpolsterelement 116 fest angebracht und ferner durch eine Umlenklasche 118 geführt, die an dem oberen Bereich 52 an dem ersten Stabilisierungselement 12 und relativ zu diesem zumindest um eine Achse drehbar angebracht ist. Ferner ist das obere Schultergurtteil 112 von der Umlenklasche 118 erneut in Richtung des Brustpolsterelements 116 zurückgeführt und mittels eines Klettverschlusses an dem Brustpolsterelement 116 und/oder an sich selbst befestig bzw. befestigbar. Abhängig von der Anbringung des Klettverschlusses kann der Abstand zwischen dem Brustpolsterelement 116 relativ zu dem oberen Bereich 52 patientenabhängig eingestellt werden.

Das untere Schultergurtteil 114 ist mit einem Ende an dem Polsterelement 28 fest angebracht. Außerdem ist das untere Schultergurtteil 114 mit seinem anderen Ende durch eine Umlenkklemmlasche 120 geführt und zu einer Schlinge 122 geformt. Die Schlinge 122 ermöglicht es einem Patienten, den unteren Schultergurtteil 114 zu greifen und den das Polsterelement 28 und die Umlenkklemmlasche 120 verbindenden Teils des unteren Schultergurtteils 114 einfach zu verkürzen. Durch Betätigung der Umlenkklemmlasche 120 kann die Länge des das Polsterelement 28 und die Umlenkklemmlasche 120 verbindenden Teils des unteren Schultergurtteils 114 erneut verlängert werden. Die Umlenkklemmlasche 120 umfasst ferner einen ersten Teil 124 eines betätigbaren Verbindungsmechanismus, der in einen an dem Brustpolsterelement 116 angebrachten zweiten Teil 126 des betätigbaren Verbindungsmechanismus angebracht bzw. anbringbar ist. An dem Brustpolsterelement 116 ist ferner ein mittleres Schultergurtteil 128 vorgesehen, an dem ein erster Teil 130 eines weiteren betätigbaren Verbindungsmechanismus ausgebildet ist. Das mittlere Schultergurtteil 128 ist durch eine Umlenklasche des ersten Teils 130 geführt und mittels eines Klettverschlusses an sich selbst befestigt, wobei abhängig von der Anbringung bzw. Anhaftung des Klettverschlusses relativ zu dem mittleren Schultergurtteil 128 die Länge des das Brustpolsterelement 116 und den ersten Teil 130 verbindenden Abschnitt des mittleren Schultergurtteils einstellbar ist. Auf dem mittleren Schultergurtteil 128 ist ferner ein Polster 131 angeordnet.

Der zweite Schultergurtzug 28 ist analog zu dem ersten Schultergurtzug 26 ausgebildet, wobei das entsprechende Brustpolsterelement 132 des zweiten Schultergurtzugs 28 im Vergleich zu dem Brustpolsterelement 128 großflächiger ausgebildet ist. Außerdem umfasst der zweite Schultergurtzug 28 kein mittleres Schultergurtteil, sondern es ist ein zweiter Teil 134 des weiteren betätigbaren Verbindungsmechanismus ausgebildet, der mit dem ersten Teil 130 koppelbar ist. Der zweite Teil 134 ist direkt an einer Lasche 136 des Polsterelements 132 fest angebracht.

Das erste und das zweite Stabilisierungselement 12, 14 und die Seitenteile 20, 22 umfassen im Wesentlichen Kunststoff, Aluminium bzw. einen Faserverbundwerkstoff. Kunststoff kann Polyethylen, Polypropylen, Polycarbonat, Polyethylenterephthalat oder weitere Kunststoffe umfassen. Die längliche Stabilisierungsschiene 16 und der Stabilisierungskörper 40 umfassen metallisches Material wie Stahl, Edelstahl, Titan, eine Metalllegierung usw. oder einen Verbundwerkstoff. Verbundwerkstoff kann einen glasfaserverstärkten oder kohlenstofffaserverstärkten Kunststoff umfassen. Die Schultergurtzüge 26, 28 und die Hüftgurtanordnung 18 umfassen Textilien, wobei diese kunstfaserbasiert ausgebildet sein können.

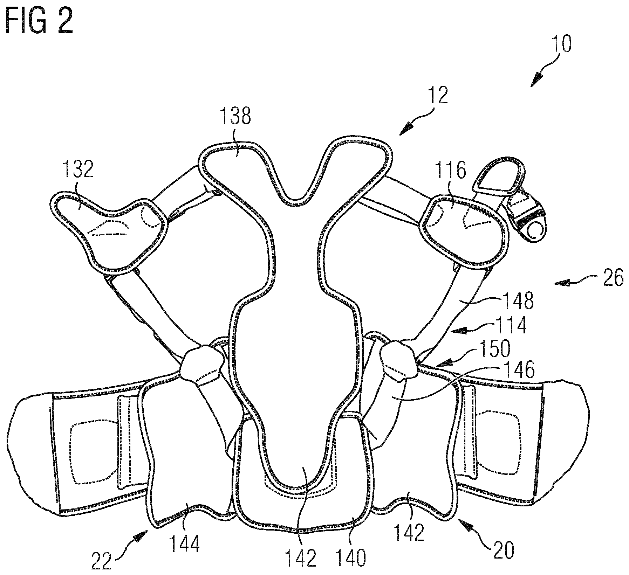

Figur 2 betrifft eine schematische Vorderansicht der Rückenorthese 10 aus Figur 1. Es ist erkennbar, dass das erste Stabilisierungselement 12 von einem flächigen Polsterelement 138 bedeckt ist, welches in einem oberen Bereich im Wesentlichen der Form des ersten Stabilisierungselements 12 entspricht. Das flächige Polsterelement 138 ragt in einem unteren Bereich jedoch über das erste Stabilisierungselement 12 hinaus und verdeckt den Bereich der Stabilisierungsschiene 16 bzw. den Bereich zwischen den Stabilisierungselementen 12, 14 und ferner einen Teil des zweiten Stabilisierungselements 14, in dem die Stabilisierungsschiene 16 an dem zweiten Stabilisierungselement 14 angebracht ist.

Das zweite Stabilisierungselement 14 ist von einem flächigen Polsterelement 140 bedeckt, wobei das flächige Polsterelement 138 mit dem flächigen Polsterelement 140 bereichsweise überlappt. So ist eine Polsterzunge 142 des flächigen Polsterelements 138 vor dem flächigen Polsterelement 140 angeordnet, d. h. in einem am Patienten angelegten Zustand zwischen dem Patienten und dem Polsterelement 140.

Ferner sind die jeweiligen Seitenteile 20, 22 von einem jeweiligen Polsterelement 142, 144 bedeckt, sodass die Seitenteile 20, 22 über das jeweilige Polsterelement 142, 144 an dem Patienten anliegen. Die Polsterelemente 142, 144 umfassen auf ihrer Rückseite Aufnahmetaschen 146 (siehe Figur 1), die dazu eingerichtet sind, das jeweilige Seitenteil 20, 22 teilweise zu umgreifen. In anderen Worten ermöglichen es die Aufnahmetaschen 146, die Polsterelemente 142, 144 auf die Seitenteile 20, 22 aufzuspannen.

Die Polsterelemente 138, 140, 142, 144 sind vergleichsweise weich ausgestaltet und bieten einem Patienten einen angenehmen Tragekomfort. Sie umfassen vliesartiges Material bzw. ein Textil. Ferner sind die Polsterelemente 138, 140, 142, 144 und die Brustpolsterelemente 116, 132 auf ihrer Innenseite im Wesentlichen eben ausgebildet bzw. an eine Körperkontur eines Menschen angepasst.

Im Zusammenhang mit dem Schultergurtzug 26 ist erkennbar, dass das untere Schultergurtteil 114 zweiteilig ausgebildet ist. Es umfasst einen ersten Gurtabschnitt 146 und einen zweiten Gurtabschnitt 148, die über eine durch ein Polster 150 verdeckte Umlenklasche 152 miteinander verbunden sind. Das Polster 150 dient dazu, in einem am Patienten angelegten Zustand der Rückenorthese 10 zwischen der Umlenklasche 152 und dem Patienten angeordnet zu sein, um so einen angenehmen Tragekomfort zu gewährleisten.

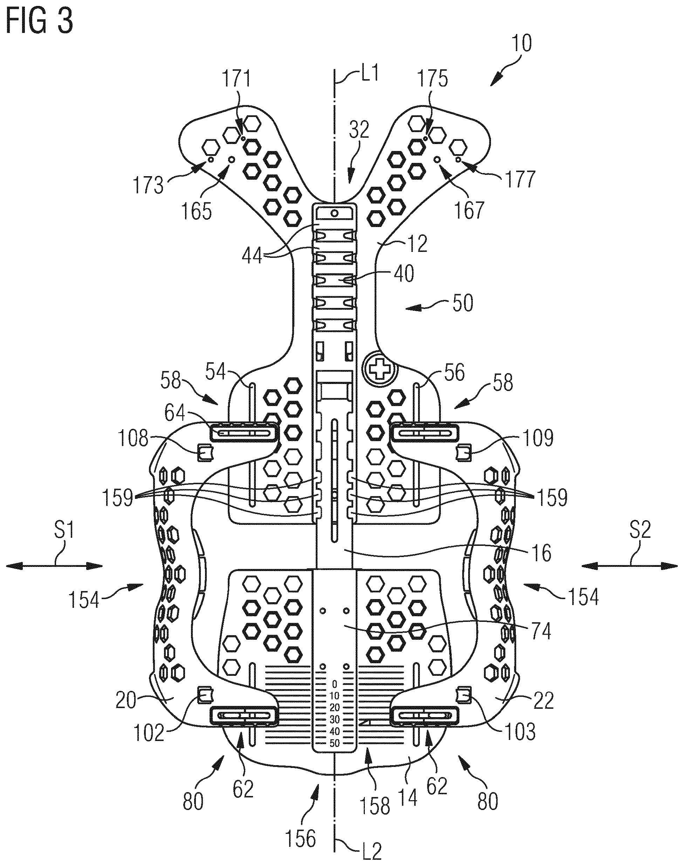

Figur 3 betrifft eine schematische, vereinfachte Rückansicht der Rückenorthese 10 gemäß dem vorangehend erläuterten Ausführungsbeispiel. Neben anderen Vereinfachungen aus Gründen der besseren Darstellung sind gegenüber Figuren 1 und 2 die sogenannten gurtartigen und stoffartigen Teile der Rückenorthese, sowie die Führungskörper in den Führungsschlitzen, einige Befestigungsmittel wie die Feststellschrauben, die Vorspanngurte usw. ausgeblendet. Diese Vereinfachungen betreffen auch die folgenden Figuren. In Figur 3 sind somit vielmehr das erste flächige Stabilisierungselement 12 und das zweite flächige Stabilisierungselement 14 dargestellt, die über die Stabilisierungsschiene 16 miteinander gekoppelt sind. Außerdem sind das erste Seitenteil 20 und das zweite Seitenteil 22 dargestellt.

Unter Bezug auf das erste Seitenteil 20 ist die Führungsanordnung 58 dargestellt, die den parallel zur Längsachse L1 ausgebildeten Führungsschlitz 54 des ersten flächigen Stabilisierungselements 12 und den an dem ersten Seitenteil 20 ausgebildeten Führungsschlitz 64 umfasst. Der Führungsschlitz 54 ermöglicht es, das erste Stabilisierungselement 12 und das erste Seitenteil 20 relativ zueinander in der Höhenrichtung bzw. entlang der Längsachse L1 zu verlagern. Ferner ermöglicht es der Führungsschlitz 64, das erste Stabilisierungselement 12 und das erste Seitenteil 20 relativ zueinander in der Seitenrichtung S1, also in Richtung medial-lateral am Patienten bzw. vorliegend senkrecht zur Längsachse L1, zu verlagern. Wie bereits erläutert ist die Führungsanordnung 58 des zweiten Seitenteils 22 relativ zu dem ersten flächigen Stabilisierungselement 12 analog ausgebildet und ermöglicht eine Verlagerung in der Seitenrichtung S2. Außerdem sind die jeweiligen Führungsanordnungen 80 betreffend das erste Seitenteil 20 relativ zu dem zweiten flächigen Stabilisierungselement 14 bzw. betreffend das zweite Seitenteil 22 relativ zu dem zweiten flächigen Stabilisierungselement 14 entsprechend analog der Führungsanordnung 58 ausgebildet und ermöglichen eine entsprechende Verlagerung in der Seitenrichtung S1 bzw. S2.

Die jeweiligen Seitenteile 20, 22 weisen eine geschwungene Form auf, die einer Körperkontur des seitlichen Bauchbereichs nachempfunden ist. Die Seitenteile 20, 22 sind derart ausgebildet, dass sie insoweit zwar formstabil sind, als dass sie eine vorgegebene Form aufweisen, jedoch sind sie elastisch verformbar, um an die Körperkontur eines jeweiligen Patienten anpassbar zu sein. Aufgrund der geschwungenen Form sind die Seitenteile 20, 22 in Bezug auf die Seitenrichtung S1 in einem oberen und einem unteren Bereich breiter als in einem mittleren Bereich ausgebildet. An den jeweiligen Seitenteilen 20, 22 ist der Spannhaken 102, 103 und der Spannhaken 108, 109 ausgebildet. Wie an den beiden Stabilisierungselementen 12, 14 sind an den Seitenteilen 20, 22 jeweils auch Materialaussparungen 154 ausgebildet.

Das zweite Stabilisierungselement 14 umfasst eine Maßskala 156 mit einer Vielzahl an horizontal bzw. senkrecht zur Längsachse L1 verlaufenden, gleichmäßig zueinander beabstandeten Maßlinien 158. Die Maßlinien 158 verlaufen entlang der Rückseite des zweiten Stabilisierungselements 14 zwischen den Bereichen, in denen die Führungsfortsätze 62 an dem zweiten Stabilisierungselement 14 anliegen bzw. an diesem angeordnet sind. Die Maßlinien 158 dienen zusammen mit der Maßskala 156 dazu, die beiden Seitenteile 20, 22 gegenüber dem zweiten Stabilisierungselement 14 und/oder relativ zu dem jeweiligen anderen Seitenteil 20, 22 auszurichten. Dies kann den Vorgang des Anpassens der Rückenorthese 10 an den Patienten erleichtert.

Wie auch in den vorangehenden Figuren 1 und 2 ist der Stabilisierungskörper 40 in der Stabilisierungsführung 32 angeordnet.

Ferner geht hervor, dass die Befestigungselemente 44 in einem oberen Bereich der Stabilisierungsführung 32 ausgebildet sind, in dem der Stabilisierungskörper 40 angeordnet ist. In einem unteren Bereich der Stabilisierungsführung 32, nämlich in einem Bereich, in dem die Stabilisierungsschiene 16 führbar bzw. vorgesehen ist, ist eine Vielzahl an Befestigungsvorsprüngen 159 beidseits und gegenüberliegend in der Stabilisierungsführung 32 und an deren rückseitigen Bereich ausgebildet. Die Befestigungsvorsprünge 159 stehen in Richtung aufeinander von den Seiten der Stabilisierungsführung 32 hervor und dienen dazu, die Stabilisierungsschiene 16 in der Stabilisierungsführung 32, insbesondere nach hinten hin, zu fixieren.

Gemäß einer Ausführungsform kann ein erstes Stabilisierungselement vorgesehen sein, das ähnlich wie das Stabilisierungselement 12 ausgebildet ist, jedoch einen kürzeren oder längeren mittleren Bereich 50 in Richtung der Längsrichtung L1 umfasst. Entsprechend der Länge des mittleren Bereichs und der Länge der Stabilisierungsführung 32 ist der Stabilisierungskörper 40 entsprechend verkürzt oder verlängert auszubilden. Anhand der Ausgestaltung der Länge des mittleren Bereichs 50 kann die Rückenorthese 10 an unterschiedliche Körpergrößen und/oder Anatomien von Patienten angepasst werden. Ferner kann die Stabilisierungswirkung der Rückenorthese 10 bedarfsgerecht angepasst werden.

Wie bereits in Zusammenhang mit den vorangehenden Figuren erläutert, sind die beiden Schultergurtzüge 26, 28 dazu vorgesehen, an ihrem jeweiligen einen Ende mit dem ersten Stabilisierungselement 12 gekoppelt zu werden. An dem ersten Stabilisierungselement 12 sind zur Kopplung mit dem ersten Schultergurtzug 26 ein erstes Durchgangsloch 165 und zur Kopplung mit dem zweiten Schultergurtzug 28 ein zweites Durchgangsloch 167 ausgebildet. Ferner sind an der vorliegend gezeigten Rückseite des Stabilisierungselements 12 und beabstandet zu jedem der Durchgangslöcher 165, 167 jeweils zwei Erhebungen 171, 173, 175, 177 ausgebildet. Die beiden Erhebungen 171, 173 sind beispielsweise auf einer imaginären Kreislinie um das Durchgangsloch 165 angeordnet und dienen dazu, die Bewegung der Umlenklasche 118 um die Achse des Durchgangslochs 165 zu begrenzen. Entsprechend sind die Erhebungen 175, 177 auf einer imaginären Kreislinie um das Durchgangsloch 167 angeordnet und dienen dazu, die Bewegung der Umlenklasche des zweiten Schultergurtzugs 28 um die Achse des Durchgangslochs 167 zu begrenzen. Genauer gesagt wird einer jeweiligen Umlenklasche ein Winkelbereich vorgegeben, innerhalb dessen die Umlenklasche jeweils um die Achse des jeweiligen Durchgangslochs 165, 167 relativ zu dem ersten Stabilisierungselement 12 verschwenkbar ist. Eine Verdrehung der jeweiligen Umlenklasche nach außerhalb dieses Winkelbereichs ist durch ein dann auftretendes Anliegen der Umlenklasche 118 an einer der Erhebungen 171, 173, 175, 177 gesperrt. Gegebenenfalls ist es mittels erhöhten Kraftaufwands, beispielsweise durch elastische Verformung der Umlenklasche 118, möglich, zumindest einer der Erhebungen 171, 173, 175, 177 zu umgehen und so den Winkelbereich zumindest vorübergehend zu verlassen. Bei normaler Nutzung verbleiben die Umlenklaschen jedoch in einer Stellung innerhalb des vorgegebenen Winkelbereichs. Der vorgegebene Winkelbereich entspricht dabei einem Winkelbereich, in dem die jeweilige Umlenklasche typischerweise während des Tragens der Rückenorthese 10 am Patienten relativ zu dem ersten Stabilisierungselement 12 ausgerichtet ist. Die Erhebungen 171, 173, 175, 177 wirken also einem Verdrehen der Umlenklaschen aus diesem Winkelbereich hinaus entgegen, was somit eine Verdrehung des Schultergurts 26, 28 verhindert. Gerade das Anlegen der Rückenorthese 10 wird aufgrund des gewährleisteten günstigen Winkelbereichs bzw. Orientierung der Umlenklaschen und damit auch der günstigen Ausrichtung des Schultergurts 26, 28 deutlich erleichtert. Der Winkelbereich kann 90 Grad oder weniger, bevorzugt 60 Grad oder weniger und besonders bevorzugt 45 Grad oder 30 Grad oder weniger betragen.

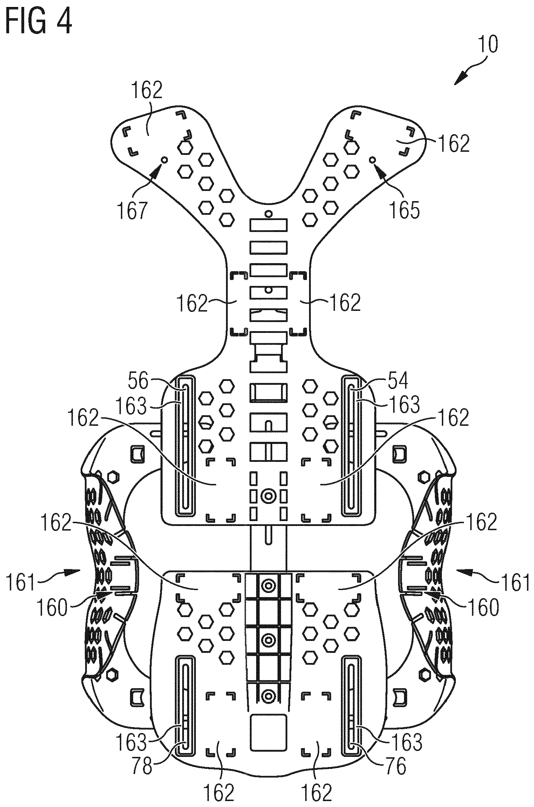

Figur 4 betrifft eine schematische, vereinfachte Vorderansicht der Rückenorthese 10 gemäß dem vorangehend erläuterten Ausführungsbeispiel. Analog zu Figur 3 sind die sogenannten gurtartigen und stoffartigen Teile der Rückenorthese 10 ausgeblendet.

Zusätzlich zur Figur 3 geht hervor, dass an den Seitenteilen 20, 22 jeweils flexible Bereiche 160 ausgebildet sind. Die flexiblen Bereiche 160 sind an dem dem ersten und zweiten flächigen Stabilisierungselement 12, 14 abgewandten Ende der Seitenteile 20, 22 ausgebildet. Die flexiblen Bereiche 160 umfassen dabei jeweils unterschiedlich tiefe Einschnitte bzw. Materialaussparungen 161, die ausgehend von dem Randbereich bzw. dem vorderen Ende des jeweiligen Seitenteils 20, 22 in dieses hinein erstreckend ausgebildet sind. Dadurch ergeben sich fächerartig breitere Materialstege, die leichter verformbar sind. Die flexiblen Bereiche 160 bewirken eine gesteigerte elatische Verformbarkeit des Seitenteils, beispielsweise zur besseren Anpassung an bestimmte Bauchumfänge von Patienten. Ferner kennt man in Figur 3 auch die hexagonalen Materialaussparungen, die in Form von Durchbrüchen oder zum Teil nur in Form von lokalen Vertiefungen ausgebildet sind.

Ferner gehen aus Figur 4 hervorgehobene Montagebereiche 162 an den beiden Stabilisierungselementen 12, 14 hervor. Dabei ist vorgesehen, an den Montagebereichen 162 jeweilige Verbindungsmittel, beispielsweise Klebstoff und/oder Klettverschlussmittel, anzubringen, um an den Stabilisierungselementen 12, 14 die Polsterelemente 138, 140 anzubringen. Die Montagebereiche 162 erleichtern es, die Rückenorthese 10 zu montieren.

Im Bereich der Führungsschlitze 54, 56 des ersten Stabilisierungselements 12 und der Führungsschlitze 76, 78 sind jeweilige Führungsvertiefungen 163 entsprechend den Führungsvertiefungen 72 ausgebildet. Diese dienen dazu, einen jeweiligen Führungskörper wie den Führungskörper 70 entlang der Führungsschlitze 54, 56, 76, 78 zu führen und mit der jeweiligen Feststellschraube 68 in Eingriff zu sein.

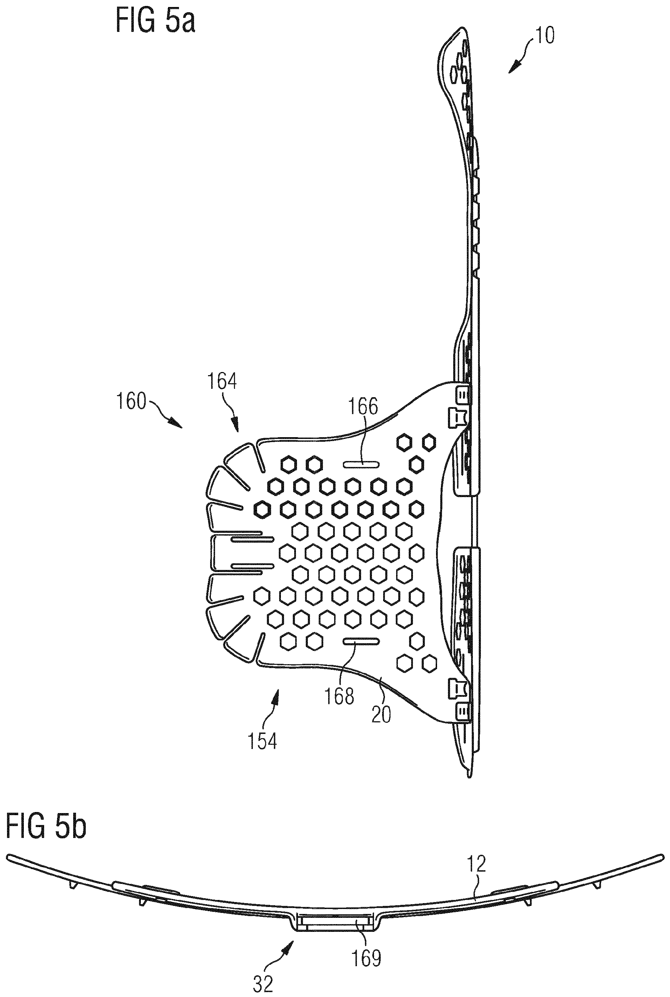

Figur 5a betrifft eine schematische vereinfachte Seitenansicht der Rückenorthese 10 gemäß dem vorangehenden Ausführungsbeispiel. Es ist mit Bezug auf das erste Seitenteil 20 erkennbar, dass eine Vielzahl der Materialaussparungen 154 an dem Seitenteil 20 ausgebildet ist. Benachbarte Materialaussparungen 154 sind zueinander im Wesentlichen gleichmäßig beanstandet angeordnet. Ferner ist im Zusammenhang mit Figur 5a die Ausprägung der flexiblen Bereiche 160 gut erkennbar. So sind die flexiblen Bereiche 160 an einem vorderen Ende 164 des Seitenteils 20 ausgebildet und erstrecken sich mit Bezug auf einen Patienten von anterior nach posterior über einen Bereich des Seitenteils von etwa 20 %.

An dem Seitenteil 20 sind ferner Aufnahmeschlitze 166, 168 ausgebildet. Diese dienen der Kopplung mit der Hüftgurtanordnung 18, insbesondere mit dem ersten Hüftgurtteil 84. Insbesondere kann eine Gurtanordnung bzw. vorangehend genannte Lasche vorgesehen sein, die durch die beiden Aufnahmeschlitze 166, 168 geführt ist. In dieser Gurtanordnung kann das erste Hüftgurtteil 84 oder eine an dem Hüftgurtteil 84 vorgesehene Gurtführungsanordnung bzw. vorangehend genannte Lasche führbar sein. Das zweite Seitenteil 22 ist analog dazu ausgebildet.

Es kann vorgesehen sein, dass die Seitenteile 20, 22 jeweils bezüglich einer Mittelebene, die beispielsweise zwischen den Aufnahmeschlitzen 166, 168 angeordnet ist, spiegelsymmetrisch aufgebaut sind.

Figur 5b betrifft eine schematische vereinfachte Untersicht des ersten Stabilisierungselements 12 gemäß dem vorangehend erläuterten Ausführungsbeispiel. Es ist erkennbar, dass an dem ersten Stabilisierungselement 12 die Stabilisierungsführung 32 ausgebildet ist, in der die Stabilisierungsschiene 16 aufnehmbar und relativ zu dem ersten Stabilisierungselement 12 führbar ist. Ferner ist je nach Ausgestaltung des ersten Stabilisierungselements 12 bzw. je nach Bedarf auch der Stabilisierungskörper 40 in der Stabilisierungsführung 32 aufnehmbar bzw. anordenbar. Außerdem bildet die Stabilisierungsführung 32 eine nach unten offene Aufnahmeöffnung 169, durch die der Stabilisierungskörper 40 und/oder die Stabilisierungsschiene 16 in die Stabilisierungsführung 32 führbar ist.

Ferner weist das erste Stabilisierungselement 12 eine um die Längsachse L1 gebogene Form auf, und zwar eine in einem am Patienten angelegten Zustand der Rückenorthese 10 um den Körper des Patienten gebogen Form. Damit ist das erste Stabilisierungselement 12 an die Körperkontur eines menschlichen Körpers zumindest teilweise angepasst. Die gebogene Form verbessert damit den Tragekomfort und erhöht die Stabilisierungswirkung der Rückenorthese 10. Das zweite Stabilisierungselement 14 kann eine ähnlich gebogene Form aufweisen. Zumindest die innere geometrische Ausgestaltung der Aufnahmetasche 74 kann ähnlich zu der Stabilisierungsführung 32 ausgebildet sein.

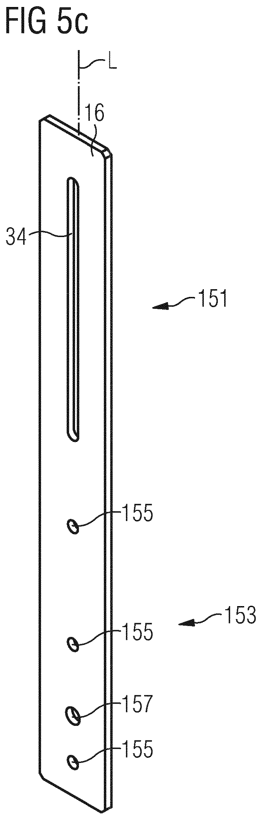

Figur 5c betrifft eine schematische vereinfachte isometrische Ansicht der Stabilisierungsschiene 16 gemäß dem vorangehend erläuterten Ausführungsbeispiel. Die Stabilisierungsschiene 16 weist in einem oberen Bereich 151 den Längsschlitz 34 auf, der sich durch die Stabilisierungsschiene 16 und entlang der Längsrichtung L erstreckt. Der Längsschlitz 34 weist eine konstante Breite auf. In einem unteren Bereich 153 ist die Stabilisierungsschiene 16 ohne einen Längsschlitz ausgebildet. Allerdings sind im unteren Bereich 153 mehrere Durchgangslöcher 155, 157 ausgebildet, wobei das Durchgangsloch 157 einen größeren Durchmesser aufweist also die drei Durchgangslöcher 155. Die Durchgangslöcher 155, 157 dienen dazu, die Stabilisierungsschiene 16 fest an dem zweiten Stabilisierungselement 14 anzuordnen, beispielsweise mittels Verschraubung, Verklebung und/oder mittels Umspritzung der Stabilisierungsschiene 16 mit dem zweiten Stabilisierungselement 14. Die Stabilisierungsschiene 16 weist eine rechteckige Grundform mit einer vorgegebenen, konstanten Materialdicke auf. In anderen Worten entspricht die Stabilisierungsschiene 16 einem dünnen, langgestreckten Quader. Abhängig davon, mit welchem der Stabilisierungselemente 12, 14 die Stabilisierungsschiene 16 fest gekoppelt ist, kann das Stabilisierungselement 16 auch umgekehrt bezüglich der Höhenrichtung orientiert in der Rückenorthese 10 vorgesehen sein. Ferner kann der Längsschlitz 34 auch entlang im Wesentlichen beider Bereiche 151, 153 der Stabilisierungsschiene 16 ausgebildet sein.

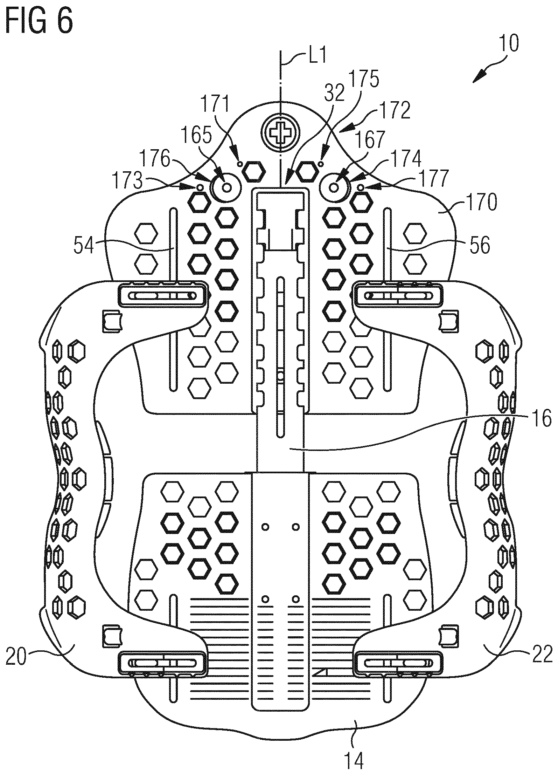

Figur 6 betrifft eine schematische vereinfachte Rückansicht der Rückenorthese 10 gemäß einem weiteren Ausführungsbeispiel. Bei diesem Ausführungsbeispiel ist die Rückenorthese 10 wie vorangehend erläutert ausgebildet, jedoch mit der Einschränkung, dass ein alternatives erstes Stabilisierungselement 170 vorgesehen ist. Das erste Stabilisierungselement 170 ist im Wesentlichen analog zu dem unteren Bereich 48 des vorangehend beschriebenen Stabilisierungselements 12 ausgebildet. So weist das Stabilisierungselement 170 die Führungsschlitze 54, 56 auf, die parallel zur Längsachse L1 ausgebildet sind. Ferner ist an dem ersten Stabilisierungselement 170 die Stabilisierungsführung 32 ausgebildet, die dazu ausgebildet ist, die Stabilisierungsschiene 16 aufzunehmen, sodass das Stabilisierungselement 170 und die Stabilisierungsschiene 16 relativ zueinander verlagerbar sind. Gegenüber dem ersten Stabilisierungselement 12 ist jedoch die Stabilisierungsführung 32 in Richtung der Längsachse L1, bzw. in Richtung eines oberen Bereichs 172 kürzer ausgebildet. In anderen Worten ist die Stabilisierungsführung 32 in dem oberen Bereich 172 nicht ausgebildet bzw. vorgesehen. Insofern ist neben der Stabilisierungsschiene 16 kein weiteres stabilisierendes Bauteil und damit auch kein Stabilisierungskörper 40 wie vorangehend beschrieben in der Stabilisierungsführung 32 geführt und/oder angeordnet.

Auch das erste Stabilisierungselement 170 ist an die Form eines Menschen Körpers, insbesondere eines mittleren und/oder oberen Rückenbereichs, angepasst. Zur Anbringung der nicht dargestellten Schultergurtanordnung sind an dem ersten Stabilisierungselement 170 entsprechende Aufnahmeöffnungen 174, 176, insbesondere Durchgangslöcher, vorgesehen, an denen die Umlenklachse 118 anbringbar ist. Wie bereits vorangehend erläutert sind an dem ersten Stabilisierungselement 170 ebenfalls die Erhebungen 171, 173, 175, 177 an dessen Rückseite ausgebildet. Ferner bildet der obere Bereich 172 einen erweiterten Auflagebereich in Richtung cranial in einem am Patienten angebrachten Zustand der Rückenorthese.

Das erste Stabilisierungselement 170 ist in der Höhenrichtung kürzer ausgebildet als das erste Stabilisierungselement 12. Der obere Bereich 172 ist nun im Bereich der Längsachse L1 höher ausgebildet als abseits der Längsachse L1.

Wie bereits erläutert, sind das erste Seitensteil 20, das zweite Seitenteil 22, das zweite Stabilisierungselement 14 und die Stabilisierungsschiene 16 wie im Zusammenhang mit Figuren 1 bis 5b erläutert ausgebildet.

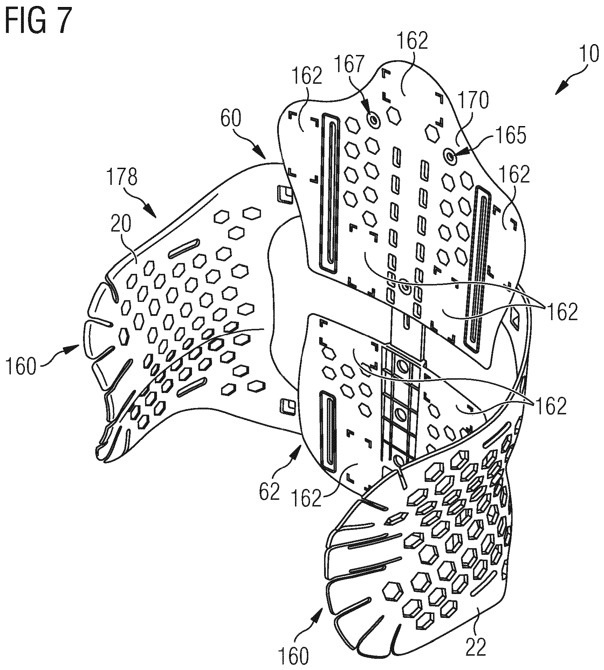

Figur 7 betrifft eine schematische vereinfachte isometrische Ansicht der Rückenorthese 10 aus Figur 6. An dem ersten Stabilisierungselement 170 sind auf dessen Innenseite wie auch bei dem Stabilisierungselement 12 hervorgehobene Montagebereiche 162 ausgebildet.

Hinsichtlich der Seitenteile 20, 22 geht aus Figur 7 erneut deren geschwungene Form hervor. So sind die Seitenteile 20, 22 jeweils in ihrem mittleren Bereich in Richtung medial eines Patienten eingewölbt und in einem unteren und oberen Bereich jeweils in Richtung lateral eines Patienten ausgewölbt ausgebildet. Ferner sind die flexiblen Bereiche 160 an einem Bereich der Seitenteile 20, 22 ausgebildet, der dazu eingerichtet ist, einen lateralen und/oder ventralen Bereich eines Patienten zu kontaktieren. Allgemein kann die Form eines Seitenteils 20, 22 als gebogene Gabelform beschrieben werden, wobei mit Bezug auf das erste Seitenteil 20 die Gabelspitzen bzw. -zinken durch den oberen Führungsfortsatz 60 und den unteren Führungsfortsatz 62 gebildet sind. Die Führungsfortsätze 60, 62 sind gegenüber einem mittleren Bereich 178 des ersten Seitenteils 20 in einem Winkel von etwa 90° ausgerichtet.

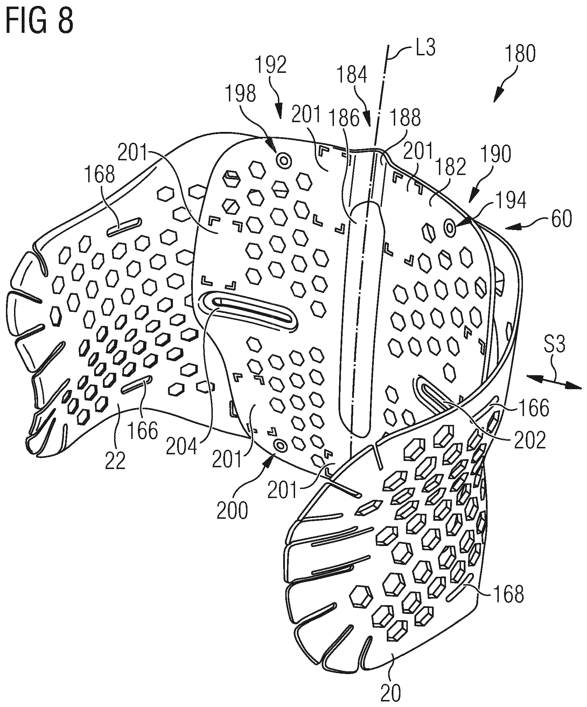

Figur 8 betrifft eine schematische vereinfachte isometrische Ansicht einer Rückenorthese 180 gemäß einem weiteren Ausführungsbeispiel. Die Rückenorthese 180 basiert auf der vorangehend beschriebenen Rückenorthese 10. Allerdings umfasst die Rückenorthese 180 lediglich ein einteiliges flächiges Stabilisierungselement 182, das dazu ausgebildet ist, den Rücken eines Patienten zu stützen.

Das mit dem Stabilisierungselement 182 gekoppelte erste Seitenteil 20 entspricht dem vorangehend beschriebenen Seitenteil 20 und das zweite Seitenteil 22 entspricht dem vorangehend beschriebenen Seitenteil 22.

Das flächige Stabilisierungselement 182 weist einen mittleren Bereich 184 auf, der sich in einem am Patienten angelegten Zustand der Rückenorthese 180 von oben nach unten und somit entlang einer Längsachse L3 erstreckt. Der mittlere Bereich 184 weist eine flächige Materialaussparung 186 und eine obere Auswölbung 188 auf. Die Materialaussparung 186 und die obere Auswölbung 188 dienen dazu, in einem am Patienten angelegten Zustand einen Bereich, in dem Wirbel des Patienten angeordnet sind, weitestgehend auszusparen, um somit eine Krafteinleitung durch die Rückenorthese 180 in den Körper des Patienten außerhalb des Wirbelbereichs zu ermöglichen. Abseits des mittleren Bereichs 184 ist ein erster Stabilisierungsbereich 190 und ein zweiter Stabilisierungsbereich 192 ausgebildet. Die beiden Stabilisierungsbereiche 190, 192 dienen dazu, in einem am Patienten angelegten Zustand einen hüftnahen Bereich des Patienten zu kontaktieren. In dem ersten Stabilisierungsbereich 190 ist eine obere Aufnahmeöffnung 194 und eine vorliegend durch das erste Seitenteil 20 verdeckte untere Aufnahmeöffnung 196 ausgebildet. Außerdem ist an dem zweiten Stabilisierungsbereich 192 ebenfalls eine obere Aufnahmeöffnung 198 und eine untere Aufnahmeöffnung 200 ausgebildet. Die Aufnahmeöffnungen 194, 196, 198, 200, die als Durchgangsöffnungen ausgebildet sind, dienen dazu, das Stabilisierungselement 182 mit dem jeweiligen Seitenteil 20, 22 zu koppeln. Dazu kann eine Verbindungsmittelanordnung wie eine Schraubenanordnung in den jeweiligen Aufnahmeöffnungen 194, 196, 198, 200 vorgesehen sein. Die Verbindungsanordnung kann analog der Führungsanordnung 58 ausgebildet sein und die Feststellschraube 68 und den Führungskörper 70 umfassen, der in dem Führungsschlitz 64 des Führungsfortsatzes 60 angeordnet und darin geführt ist. In einem gelockerten Zustand der Verbindungsanordnung kann das jeweilige Seitenteil 20, 22 relativ zu dem Stabilisierungselement 182 in Richtung medial - laterial verlagert werden, während in einem angezogenen Zustand der Verbindungsanordnungen in den Aufnahmeöffnungen 194, 196 bzw. 198, 200 das jeweilige Seitenteil 20, 22 relativ zu dem Stabilisierungselement 182 fest angeordnet ist.

An den Seitenteilen 20, 22 sind die Aufnahmeschlitze 166, 168 ausgebildet. Diese dienen dazu, eine Hüftgurtanordnung wie beispielsweise die Hüftgurtanordnung 18 aus Figuren 1 und 2 mit dem jeweiligen Seitenteil 20, 22 wie vorangehend erläutert zu koppeln. Somit kann eine einfache Rückenorthese 180 bereitgestellt werden, die insbesondere für einen hüftnahen Bereich eines Patienten vorgesehen ist.

Im vorderen Bereich des Stabilisierungselements 182 ist eine Mehrzahl an Montagebereichen 201 vorgesehen, die wie die Montagebereiche 162 dazu vorgesehen sind, jeweilige Verbindungsmittel, beispielsweise Klebstoff und/oder Klettverschlussmittel, anzubringen, um an dem Stabilisierungselement 182 ein nicht dargestelltes Polsterelement anzubringen, das im Wesentlichen die Form des Stabilisierungselements 182 aufweist und der Polsterung gegenüber dem Patienten dient. Das Polsterelement kann insbesondere bezüglich Material wie die Polsterelemente 138, 140, 142, 144 ausgebildet sein.

An dem Stabilisierungselement 182 ist ferner ein erster Führungsschlitz 202 und ein zweiter Führungsschlitz 204 ausgebildet, wobei die Führungsschlitze 202, 204 in Richtung medial - lateral bzw. senkrecht zur Längsachse L3 und damit in einer Seitenrichtung S3 ausgerichtet und ausgebildet sind. Ferner sind die Führungsschlitze 202, 204 bezogen auf die Höhe des Stabilisierungselements 182 (in Richtung der Längsachse L3) in einem mittleren Bereich ausgebildet. Die Führungsschlitze 202, 204 dienen dazu, die vorangehend beschriebene Hüftgurtanordnung 18 an dem Stabilisierungselement 182 statt und/oder zusätzlich zu der Anbringung an den Aufnahmeschlitzen 166, 168 anzubringen. Auf diese Weise kann eine weitere Rückenorthese gemäß einem weiteren Ausführungsbeispiel bereitgestellt werden. Bei dieser Rückenorthese kann auf die beiden Seitenteile 20, 22 verzichtet werden, und eine Hüftgurtanordnung wie die Hüftgurtanordnung 18 direkt an dem Stabilisierungselement 182 angebracht werden. Dazu kann eine Verbindungsmittelanordnung wie beispielsweise eine Schraubenanordnung vorgesehen sein, wobei dann beispielsweise eine jeweilige Feststellschraube durch das Stabilisierungselement 182, genauer gesagt in dem jeweiligen Führungsschlitz 202, 204, angeordnet wird. Die Feststellschraube ist ferner mit einer zugehörigen Schraubenaufnahme 206, 208 (siehe Figur 1), der Hüftgurtanordnung 18 koppelbar bzw. gekoppelt, wobei als Schraubenaufnahme 206, 208 beispielsweise eine an der Hüftgurtanordnung vorgesehene Gewindehülse vorgesehen sein kann. In einem gelösten Zustand der jeweiligen Feststellschraube kann das zugehörige Hüftgurtteil 84, 86 relativ zu dem Stabilisierungselement 182 innerhalb der Grenzen des jeweiligen Führungsschlitzes 202, 204 verlagert werden. In einem angezogenen Zustand der jeweiligen Feststellschraube ist das entsprechende Hüftgurtteil 84, 86 relativ zu dem Stabilisierungselement 182 fest angeordnet bzw. mit diesem fest gekoppelt. Mit dem gelösten Zustand kann gemeint sein, dass die Feststellschraube zwar weiterhin mit der zugehörigen Schraubenaufnahme 206, 208 gekoppelt ist, jedoch nicht vollständig angezogen ist. Dies bewirkt, dass die Hüftgurtanordnung 18 relativ zu dem Stabilisierungselement 182 in der Höhenrichtung, also in Richtung der Längsachse L3 fest angeordnet ist, jedoch eine Verlagerung der jeweiligen Hüftgurtteile 84, 86 in Richtung medial - lateral, also senkrecht zur Längsrichtung L3, relativ zu dem Stabilisierungselement 182 in den Grenzen der Längsschlitze 202, 204 zugelassen ist. So dies ermöglicht es, die Hüftgurtanordnung 18 an einem Patienten anzulegen, nämlich indem die beiden Hüftgurtteile 84, 86 an einer Vorderseite des Patienten zusammengeführt und aneinander befestigt werden. Anschließend kann die Stabilisierungswirkung der Rückenorthese 180 durch Betätigen des Spannmechanismus 88 eingestellt werden. Häufig wird der Spannmechanismus 88 dazu verwendet werden, die Hüftgurtteile 84, 86 fest miteinander zu verspannen.

Das Stabilisierungselement 182 weist eine insbesondere bezüglich der Längsachse L3 gebogene Form auf und ist damit an die typische Form eines Rückens zumindest näherungsweise angepasst.

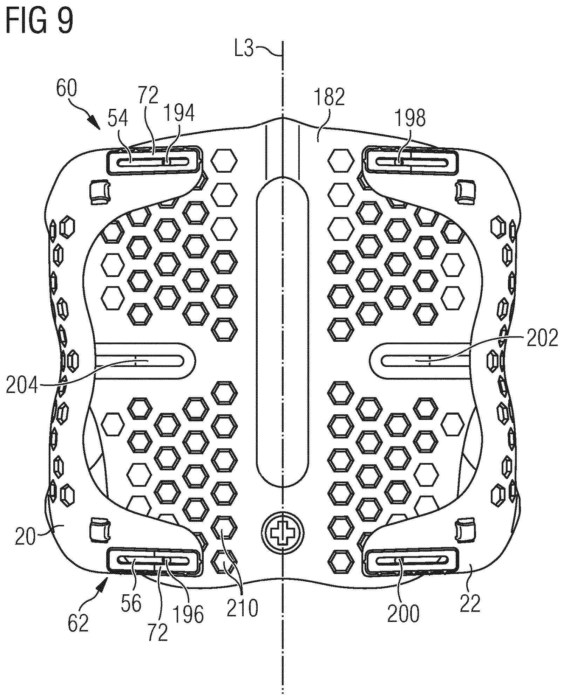

Figur 9 betrifft eine schematische vereinfachte Rückansicht der Rückenorthese 180 gemäß dem weiteren Ausführungsbeispiel gemäß Figur 8. Wie bereits vorangehend erläutert, entsprechen die Seitenteile 20, 22 den bereits erläuterten Seitenteilen 20, 22. So ist mit Bezug auf das Seitenteil 20 an dem oberen Führungsfortsatz 60 der Führungsschlitz 54 und an dem unteren Führungsfortsatz 62 der Führungsschlitz 56 ausgebildet. Ferner ist um den Führungsschlitz 54 die Führungsvertiefung 72 ausgebildet. Entsprechend ist auch um den Führungsschlitz 56 die Führungsvertiefung 72 ausgebildet.

Es ist erkennbar, dass die Führungsschlitze 54, 56 mit den Aufnahmeöffnungen 194, 196, 198, 200 überlappen, um eine Anbringung der Seitenteile 20, 22 an dem Stabilisierungselement 182 zu ermöglichen.

An dem Stabilisierungselement 182 sind jeweilige wabenförmige Materialaussparungen 210 analog zu den Materialaussparungen 82 ausgebildet, die dazu dienen, Gewicht an der Rückenorthese 180, insbesondere dem Stabilisierungselement 182, einzusparen. Ferner dienen die Materialaussparungen 210 dazu, das ästhetische Erscheinungsbild der Rückenorthese 180 zu verbessern. Die Materialaussparungen 210 sind abseits der Führungsschlitze 54, 56 und abseits der Führungsschlitze 202, 204 in dem Stabilisierungselement 182 ausgebildet.

Rein klarstellend wird darauf hingewiesen, dass die Rückenorthese 180 gemäß den Figuren 8 und 9 in den beiden Ausführungsformen, also
- mit Seitenteilen 20, 22 und Hüftgurtanordnung 18; oder
- ohne Seitenteile 20, 22, jedoch mit Hüftgurtanordnung 18;
auf eine Schultergurtanordnung wie die Schultergurtanordnung 24 verzichtet werden kann. Die Rückenorthese 180 ist dazu ausgebildet, an einem Patienten mittels der Hüftgurtanordnung 18 angebracht zu werden. Abhängig von den Beschwerden des Patienten kann die Rückenorthese 180 gemäß einer der Ausführungsformen ausgebildet sein.

Klarstellend sei angemerkt, dass die Längsrichtungen L, L1, L2 und L3 jeweils als Geraden ausgebildet sein können. Abhängig von der Ausgestaltung, einer gezielten Verformung oder Verformbarkeit der zugehörigen Bauteile kann eine jeweilige Längsrichtung jedoch auch einen gekrümmten Verlauf betreffen.

Allgemein sei angemerkt, dass alle Merkmale, die im Zusammenhang mit der Rückenorthese 10 genannt oder erläutert sind, auch im Zusammenhang mit der Rückenorthese 180 vorgesehen sein können und umgekehrt. Ferner können einzelne Merkmale, die im Zusammenhang mit einer der vorangehend beschriebenen Rückenorthesen oder einem Bauteil derselben erläutert wurden, isoliert und als eigenständige Lehre betrachtet werden.

Insgesamt wird aus den vorangehenden Erläuterungen die vielseitige Einstellbarkeit der Rückenorthese und die ebenfalls vielseitige Verwendbarkeit einzelner Teile der Rückenorthese deutlich.

## Patentansprüche

1. Rückenorthese (10; 180) zum Stabilisieren eines Rückens eines Patienten, umfassend
- ein erstes flächiges Stabilisierungselement (12) zur Stützung des Rückens des Patienten;
- ein zweites flächiges Stabilisierungselement (14) zur Stützung des Rückens des Patienten unterhalb des ersten flächigen Stabilisierungselements (12); und
- eine längliche Stabilisierungsschiene (16), die das erste und zweite flächige Stabilisierungselement (12; 14) miteinander koppelt und derart relativ zu dem ersten und/oder zweiten flächigen Stabilisierungselement (12; 14) geführt ist, dass eine geführte translatorische Verlagerung des ersten und des zweiten flächigen Stabilisierungselements (12; 14) zueinander in der Längsrichtung (L) der Stabilisierungsschiene (16) zur Anpassung der Rückenorthese (10; 180) an den Patienten möglich ist,
**gekennzeichnet durch** zumindest ein Seitenteil (20; 22) zum zumindest teilweisen Umgreifen eines lateralen Körperbereichs des Patienten, wobei das Seitenteil (20; 22) mit dem ersten flächigen Stabilisierungselement (12) und dem zweiten flächigen Stabilisierungselement (14) gekoppelt oder koppelbar ist.

2. Rückenorthese (10; 180) nach Anspruch 1, wobei die Stabilisierungsschiene (16) im Wesentlichen starr, insbesondere starr im Vergleich zu dem ersten und/oder zweiten flächigen Stabilisierungselement (12; 14), ausgebildet ist.

3. Rückenorthese (10; 180) nach einem der Ansprüche 1 oder 2, wobei die längliche Stabilisierungsschiene (16) im Wesentlichen mittig des ersten und/oder zweiten flächigen Stabilisierungselements (12; 14) angeordnet ist.

4. Rückenorthese (10; 180) nach einem der vorangehenden Ansprüche, wobei die Stabilisierungsschiene (16) fest mit einem von dem ersten oder zweiten flächigen Stabilisierungselement (12; 14) gekoppelt oder an diesem ausgebildet ist.

5. Rückenorthese (10; 180) nach einem der vorangehenden Ansprüche, wobei die Stabilisierungsschiene (16) das erste und zweite flächige Stabilisierungselement (12; 14) derart miteinander koppelt und derart relativ zu dem ersten und/oder zweiten flächigen Stabilisierungselement (12; 14) geführt ist, dass eine translatorische Verlagerung des ersten und des zweiten flächigen Stabilisierungselements (12; 14) zueinander in zumindest einer ersten zur Längsrichtung (L) der Stabilisierungsschiene (16) senkrecht ausgerichteten Richtung gesperrt und bevorzugt ferner in einer zweiten Richtung gesperrt ist, wobei besonders bevorzugt die Längsrichtung (L) und die zweite Richtung zueinander senkrecht ausgerichtet sind.

6. Rückenorthese (10; 180) nach einem der vorangehenden Ansprüche, wobei die Stabilisierungsschiene (16) ferner das erste und zweite flächige Stabilisierungselement (12; 14) derart miteinander koppelt und derart relativ zu dem ersten und/oder zweiten flächigen Stabilisierungselement (12; 14) geführt ist, dass eine rotatorische Verlagerbarkeit der flächigen Stabilisierungselemente (12; 14) zueinander gesperrt ist.

7. Rückenorthese (10; 180) nach einem der vorangehenden Ansprüche, wobei das erste und/oder zweite flächige Stabilisierungselement (12; 14) von der Stabilisierungsschiene (16) entkoppelbar ist, insbesondere durch Verlagern des Stabilisierungselements (12; 14) relativ zu der Stabilisierungsschiene (16) in der Längsrichtung (L) der Stabilisierungsschiene (16).

8. Rückenorthese (10; 180) nach einem der vorangehenden Ansprüche, ferner umfassend eine Schienenbefestigungsanordnung (36) zum Sperren der Verlagerbarkeit des ersten und zweiten flächigen Stabilisierungselements (12; 14) zueinander in der Längsrichtung (L) der Stabilisierungsschiene (16).

9. Rückenorthese (10; 180) nach einem der vorangehenden Ansprüche, ferner umfassend zumindest einen Vorspannmechanismus (100), der dazu eingerichtet ist, zwei zueinander verlagerbare Bestandteile der Rückenorthese (10; 180) relativ zueinander, insbesondere in Richtung aufeinander, mittels einer Spannkraft zu drängen.

10. Rückenorthese (10; 180) nach Anspruch 9, ferner umfassend:
eine Führungsanordnung (58; 80), die es ermöglicht, das Seitenteil (20; 22) relativ zu dem ersten und/oder zweiten flächigen Stabilisierungselement (12; 14) in einer Seitenrichtung (S1; S2) zu verlagern;
wobei der Vorspannmechanismus (100) dazu eingerichtet ist, das Seitenteil (20; 22) in der Seitenrichtung (S1; S2), insbesondere in medialer Richtung, relativ zu dem ersten und/oder zweiten flächigen Stabilisierungselement (12; 14) mittels der Spannkraft zu drängen.

11. Rückenorthese (10; 180) nach Anspruch 10, wobei die Führungsanordnung (58; 80) einen Sperrmechanismus zum Sperren der Verlagerbarkeit des Seitenteils (20; 22) relativ zu dem ersten und/oder zweiten flächigen Stabilisierungselement (12; 14) zumindest in der Seitenrichtung (S1; S2) umfasst.

12. Rückenorthese (10; 180) nach einem der Ansprüche 9 bis 11, wobei der Betrag der Spannkraft des Vorspannmechanismus (100) einstellbar ist.

13. Rückenorthese (10; 180) nach einem der Ansprüche 10 bis 12, wobei das Seitenteil (20; 22) insbesondere an dessen dem ersten und/oder zweiten flächigen Stabilisierungselement (12; 14) abgewandten Ende flexible Bereiche (160) umfasst, die eine gesteigerte elastische Verformbarkeit des Seitenteils (20; 22) bewirken, wobei bevorzugt die flexiblen Bereiche (160) Materialaussparungen (161) umfassen, die ausgehend von einem Randbereich des Seitenteils (20; 22) in dieses hinein erstreckend ausgebildet sind.

14. Rückenorthese (10; 180) nach einem der vorangehenden Ansprüche, ferner umfassend
eine insbesondere zweiteilige Hüftgurtanordnung (18), die an dem Seitenteil (20; 22) und/oder dem ersten und/oder zweiten flächigen Stabilisierungselement (12; 14) angebracht oder anbringbar ist, und dazu eingerichtet ist, einen hüftnahen Bereich des Patienten zu umgreifen,
wobei die Hüftgurtanordnung (18) einen betätigbaren Spannmechanismus (88), insbesondere einen Seilzugmechanismus, umfasst, der dazu eingerichtet ist, die beiden Teile der Hüftgurtanordnung (18) bei Betätigung zueinander, bevorzugt in Umfangsrichtung des Patienten, zu verspannen.

15. Rückenorthese (10; 180) nach einem der vorangehenden Ansprüche, ferner umfassend
eine Schultergurtanordnung (24) mit wenigstens zwei Schultergurtzügen (26; 28), wobei ein jeweiliger Schultergurtzug (26; 28) mit dessen jeweiligen einen Bereich an das erste flächige Stabilisierungselement (12) und mit dessen jeweiligen anderen Bereich an dem zweiten flächigen Stabilisierungselement (14) koppelbar oder gekoppelt ist,
und wobei jeder Schultergurtzug (26; 28) bei Anbringung am Patienten von dem ersten flächigen Stabilisierungselement (12) über eine Schulter des Patienten geführt oder führbar ist.

## Claims

1. A back orthosis (10; 180) for stabilizing a patient's back, comprising:
- a first planar stabilizing element (12) to support the patient's back;
- a second planar stabilizing element (14) to support the patient's back below the first planar stabilizing element (12); and
- an elongated stabilizing splint (16) coupling the first and second planar stabilizing elements (12; 14) to each other and being guided relative to the first and/or second planar stabilizing elements (12; 14), enabling a guided translational displacement of the first and second planar stabilizing elements (12; 14) relative to each other in the longitudinal direction (L) of the stabilizing splint (16) to adapt the back orthosis (10; 180) to the patient,
**characterized by** at least one side part (20; 22) for at least partially surrounding a patient's lateral body region, the side part (20; 22) being coupled or couplable to the first planar stabilizing element (12) and the second planar stabilizing element (14).

2. The back orthosis (10; 180) of claim 1, wherein the stabilizing splint (16) is substantially rigid, in particular rigid in comparison to the first and/or second planar stabilizing element (12; 14).

3. The back orthosis (10; 180) of claim 1 or 2, wherein the elongated stabilizing splint (16) is disposed substantially at the center of the first and/or second planar stabilizing elements (12; 14).

4. The back orthosis (10; 180) of any one of the preceding claims, wherein the stabilizing splint (16) is firmly coupled to or formed on one of the first or second planar stabilizing element (12; 14).

5. The back orthosis (10; 180) of any one of the preceding claims, wherein the stabilizing splint (16) couples the first and second planar stabilizing elements (12; 14) to each other and is guided relative to the first and/or second planar stabilizing elements (12; 14) in such a way that translational displacement of the first and second planar stabilizing elements (12; 14) relative to each other is disabled in at least a first direction perpendicular to the longitudinal direction (L) of the stabilizing splint (16) and is preferably further disabled in a second direction, wherein it is particularly preferred that the longitudinal direction (L) and the second direction are perpendicular to each other.

6. The back orthosis (10; 180) of any one of the preceding claims, wherein the stabilizing splint (16) further couples the first and second planar stabilizing elements (12; 14) to each other and is guided relative to the first and/or second planar stabilizing elements (12; 14) in such a way that rotational displacement of the planar stabilizing elements (12; 14) relative to each other is disabled.

7. The back orthosis (10; 180) of any one of the preceding claims, wherein the first and second planar stabilizing elements (12; 14) can be decoupled from the stabilizing splint (16), in particular by displacing the stabilizing element (12; 14) relative to the stabilizing splint (16) in the longitudinal direction (L) of the stabilizing splint (16).

8. The back orthosis (10; 180) of any one of the preceding claims, further comprising a splint fastening assembly (36) for disabling displacement of the first and second planar stabilizing elements (12; 14) relative to each other in the longitudinal direction (L) of the stabilizing splint (16).

9. The back orthosis (10; 180) of any one of the preceding claims, further comprising at least one pretensioning mechanism (100) configured to push two components of the back orthosis (10; 180) that are displaceable relative to each other in relation to each other, in particular toward each other, by means of a tensioning force.

10. The back orthosis (10; 180) of claim 9, further comprising:
a guide assembly (58; 80) enabling displacement of the side part (20; 22) relative to the first and/or second planar stabilizing elements (12; 14) in a lateral direction (S1; S2);
wherein the pretensioning mechanism (100) is configured to push the side part (20; 22) in the lateral direction (S1; S2), in particular in a medial direction, relative to the first and/or second planar stabilizing elements (12; 14) by means of the tensioning force.

11. The back orthosis (10; 180) of claim 10, wherein the guide assembly (58; 80) comprises a locking mechanism for disabling displacement of the side part (20; 22) relative to the first and/or second planar stabilizing elements (12; 14) at least in the lateral direction (S1; S2).

12. The back orthosis (10; 180) of any one of claims 9 to 11, wherein the amount of the tensioning force of the pretensioning mechanism (100) is adjustable.

13. The back orthosis (10; 180) of any one of claims 10 to 12, wherein the side part (20; 22) comprises flexible areas (160), in particular on its end facing away from the first and/or second planar stabilizing elements (12; 14), which cause increased elastic deformability of the side part (20; 22), wherein the flexible areas (160) preferably comprise material cutouts (161) extending from an edge area of and into the side part (20; 22).

14. The back orthosis (10; 180) of any one of the preceding claims, further comprising:
a two-piece hip strap assembly (18), in particular, attached or attachable to the side part (20; 22) and/or the first and/or second planar stabilizing elements (12; 14) and configured to surround a patient's region close to their hips,
wherein the hip strap assembly (18) comprises an operable tensioning mechanism (88), in particular a pulley mechanism, configured to tension the two pieces of the hip strap assembly (18) upon operation relative to each other, preferably in the circumferential direction of the patient.

15. The back orthosis (10; 180) of any one of the preceding claims, further comprising:
a shoulder strap assembly (24) comprising at least two shoulder pull straps (26; 28), wherein a respective shoulder pull strap (26; 28) is couplable or coupled to the first planar stabilizing element (12) with its respective one area and to the second planar stabilizing element (14) with its respective other area,
and wherein each shoulder pull strap (26; 28), when being attached to the patient, is guided or guidable from the first planar stabilizing element (12) over a patient's shoulder.

## Revendications

1. Orthèse dorsale (10 ; 180) pour stabiliser le dos d'un patient, comprenant :
- un premier élément de stabilisation plat (12) destiné à soutenir le dos du patient ;
- un deuxième élément de stabilisation plat (14) destiné à soutenir le dos du patient sous le premier élément de stabilisation plat (12) ; et
- un profilé de stabilisation allongé (16) qui couple entre eux le premier et le deuxième élément de stabilisation plat (12 ; 14) et qui est guidé par rapport au premier et/ou au deuxième élément de stabilisation plat (12 ; 14) de telle sorte qu'un déplacement en translation guidé du premier et du deuxième élément de stabilisation plat (12 ; 14) l'un par rapport à l'autre dans la direction longitudinale (L) du profilé de stabilisation (16) soit possible afin d'adapter l'orthèse dorsale (10 ; 180) au patient,
**caractérisée par** au moins une partie latérale (20 ; 22) destinée à entourer au moins partiellement une zone latérale du corps du patient, la partie latérale (20 ; 22) étant couplée ou pouvant être couplée au premier élément de stabilisation plat (12) et au deuxième élément de stabilisation plat (14).

2. Orthèse dorsale (10 ; 180) selon la revendication 1, dans laquelle le profilé de stabilisation (16) est essentiellement rigide, en particulier rigide par rapport au premier et/ou au deuxième élément de stabilisation plat (12 ; 14).

3. Orthèse dorsale (10 ; 180) selon l'une des revendications 1 ou 2, dans laquelle le profilé de stabilisation allongé (16) est disposé essentiellement au centre du premier et/ou du deuxième élément de stabilisation plat (12 ; 14).

4. Orthèse dorsale (10 ; 180) selon l'une des revendications précédentes, dans laquelle le profilé de stabilisation (16) est couplé de manière fixe à l'un du premier ou du deuxième élément de stabilisation plat (12 ; 14) ou est formé sur celui-ci.

5. Orthèse dorsale (10 ; 180) selon l'une des revendications précédentes, dans laquelle le profilé de stabilisation (16) couple entre eux le premier et le deuxième élément de stabilisation plat (12 ; 14) et est guidé par rapport au premier et/ou au deuxième élément de stabilisation plat (12 ; 14) de telle sorte qu'un déplacement en translation du premier et du deuxième élément de stabilisation plat (12 ; 14) l'un par rapport à l'autre dans au moins une première direction orientée perpendiculairement à la direction longitudinale (L) du profilé de stabilisation (16) soit bloqué et de préférence également bloqué dans une deuxième direction, la direction longitudinale (L) et la deuxième direction étant particulièrement de préférence orientées perpendiculairement l'une par rapport à l'autre.

6. Orthèse dorsale (10 ; 180) selon l'une des revendications précédentes, dans laquelle le profilé de stabilisation (16) couple en outre entre eux le premier et le deuxième élément de stabilisation plat (12 ; 14) et est guidé par rapport au premier et/ou au deuxième élément de stabilisation plat (12 ; 14) de telle sorte qu'une possibilité de déplacement en rotation des éléments de stabilisation plats (12 ; 14) l'un par rapport à l'autre soit bloquée.

7. Orthèse dorsale (10 ; 180) selon l'une des revendications précédentes, dans laquelle le premier et/ou le deuxième élément de stabilisation plat (12 ; 14) peut être découplé du profilé de stabilisation (16), en particulier par déplacement de l'élément de stabilisation (12 ; 14) par rapport au profilé de stabilisation (16) dans la direction longitudinale (L) du profilé de stabilisation (16).

8. Orthèse dorsale (10 ; 180) selon l'une des revendications précédentes, comprenant en outre un dispositif de fixation de profilé (36) pour bloquer la possibilité de déplacement du premier et du deuxième élément de stabilisation plat (12 ; 14) l'un par rapport à l'autre dans la direction longitudinale (L) du profilé de stabilisation (16).

9. Orthèse dorsale (10 ; 180) selon l'une des revendications précédentes, comprenant en outre au moins un mécanisme de précontrainte (100) qui est conçu pour pousser l'un par rapport à l'autre, en particulier l'un vers l'autre, deux composants de l'orthèse dorsale (10 ; 180) déplaçables l'un par rapport à l'autre, au moyen d'une force de tension.

10. Orthèse dorsale (10 ; 180) selon la revendication 9, comprenant en outre :
un dispositif de guidage (58 ; 80) qui permet de déplacer la partie latérale (20 ; 22) par rapport au premier et/ou au deuxième élément de stabilisation plat (12 ; 14) dans une direction latérale (S1 ; S2) ;
dans laquelle le mécanisme de précontrainte (100) est conçu pour pousser la partie latérale (20 ; 22) dans la direction latérale (S1 ; S2), en particulier dans la direction médiale, par rapport au premier et/ou au deuxième élément de stabilisation plat (12 ; 14) au moyen de la force de tension.

11. Orthèse dorsale (10 ; 180) selon la revendication 10, dans laquelle le dispositif de guidage (58 ; 80) comprend un mécanisme de blocage pour bloquer la possibilité de déplacement de la partie latérale (20 ; 22) par rapport au premier et/ou au deuxième élément de stabilisation plat (12 ; 14) au moins dans la direction latérale (S1 ; S2).

12. Orthèse dorsale (10 ; 180) selon l'une des revendications 9 à 11, dans laquelle la valeur de la force de tension du mécanisme de précontrainte (100) est réglable.

13. Orthèse dorsale (10 ; 180) selon l'une des revendications 10 à 12, dans laquelle la partie latérale (20 ; 22) comprend, en particulier à son extrémité opposée au premier et/ou au deuxième élément de stabilisation plat (12 ; 14), des zones flexibles (160) qui augmentent la déformabilité élastique de la partie latérale (20 ; 22), les zones flexibles (160) comprenant de préférence des évidements de matériau (161) qui sont formés à partir d'une zone de bord de la partie latérale (20 ; 22) et s'étendent à l'intérieur de celle-ci.

14. Orthèse dorsale (10 ; 180) selon l'une des revendications précédentes, comprenant en outre :
un dispositif de ceinture abdominale (18) en particulier en deux parties, qui est fixé ou peut être fixé à la partie latérale (20 ; 22) et/ou au premier et/ou au deuxième élément de stabilisation plat (12 ; 14), et qui est conçu pour entourer une zone proche des hanches du patient,
dans laquelle le dispositif de ceinture abdominale (18) comprend un mécanisme de serrage actionnable (88), en particulier un mécanisme à câbles, qui est conçu pour serrer les deux parties du dispositif de ceinture abdominale (18) l'une vers l'autre, de préférence dans la direction circonférentielle du patient, lorsqu'il est actionné.

15. Orthèse dorsale (10 ; 180) selon l'une des revendications précédentes, comprenant en outre :
un dispositif de sangles d'épaule (24) avec au moins deux sangles d'épaule (26 ; 28), une sangle d'épaule respective (26 ; 28) pouvant être couplée ou étant couplée par une de ses zones respectives au premier élément de stabilisation plat (12) et par son autre zone respective au deuxième élément de stabilisation plat (14),
et dans laquelle chaque sangle d'épaule (26 ; 28), lorsqu'elle est fixée sur le patient, est guidée ou peut être guidée par le premier élément de stabilisation plat (12) par-dessus une épaule du patient.
